# EUROPÄISCHE PATENTANMELDUNG

(11) **EP 4 670 500 A2**
(43) Veröffentlichungstag der Anmeldung: **31.12.2025**
(21) Anmeldenummer: 25215835.7
(22) Anmeldetag: 23.08.2023
(51) Int. Cl.: A01K 43/00

(54) **MESSSYSTEM UND VERFAHREN ZUR NICHT-INVASIVEN IN-OVO GESCHLECHTSERKENNUNG EINES VOGELEMBRYOS IN EINEM EI IN DER FRÜH-EMBRYONALEN ENTWICKLUNG**

(30) Priorität: 29.08.2022 DE 102022121790
(62) Teilanmeldung aus: 23761811.1
(71) Anmelder: Omegga GmbH, 81671 München (DE)
(72) Erfinder: NÖLLGEN, Till, 81241 München (DE); GÜNTHER, Paul, 81477 München (DE); EDER, Moritz, 85221 Dachau (DE); HIROYASU, Kyle, 80337 München (DE)
(74) Vertreter: Meissner Bolte Partnerschaft mbB

(57) **Zusammenfassung**

Die vorliegende Erfindung bezieht sich auf ein Messsystem zur nicht-invasiven, vorzugsweise automatisierten, Geschlechtserkennung von Embryos in einem Ei 50 in der früh-embryonalen Entwicklung, insbesondere vor dem siebten Bruttag, insbesondere während der Inkubation, insbesondere in einem Inkubator mit mindestens einem Eier-Tray 40 zur Aufnahme einer Vielzahl von Eiern 50 und mindestens einem Eier-Trolley 80 zur Halterung mindestens eines Eier-Trays 40, aufweisend mindestens eine Bestrahlungseinheit 10 zur Bestrahlung eines Eis 50 mit elektromagnetischer Strahlung; mindestens eine Sensoreinheit 20 zur Erfassung von durch das Ei 50 transmittierter elektromagnetischer Strahlung; eine Auswertungseinheit 30 mit mindestens einem Spektrometer 31, das dazu ausgebildet ist, die durch das Ei 50 transmittierte Strahlung zu empfangen und ein Spektrum der durch das Ei 50 transmittierten Strahlung zu erzeugen; und einer Datenverarbeitungseinheit 33, die dazu ausgebildet ist, von dem Spektrometer 31 erzeugte Spektren zu empfangen und als Referenzspektrum oder Messspektrum abzuspeichern; eine Identifikationseinheit 23 zur Erzeugung von Identifikationsdaten, mittels derer die an einem Ei 50 erzeugten Spektren dem Ei 50 eindeutig zuordenbar sind; und eine Klassifikationseinheit 35;
wobei die Datenverarbeitungseinheit 33 dazu ausgebildet ist, von dem Spektrometer 31 erzeugte Spektren und zugehörige Identifikationsdaten zu speichern, und wobei die Klassifikationseinheit 35 dazu ausgebildet ist, das Geschlecht des Embryos auf Grundlage mindestens eines Referenzspektrums und mindestens eines Messspektrums zu bestimmen. Im Rahmen der Erfindung wird ferner ein entsprechendes Verfahren angegeben.

## Beschreibung

Die vorliegende Erfindung bezieht sich auf ein Messsystem zur nicht-invasiven, vorzugsweise automatisierten, Geschlechtserkennung von Embryos im Ei in der frühembryonalen Entwicklung, insbesondere vor dem siebten Bruttag, insbesondere während der Inkubation gemäß dem Gegenstand von Anspruch 1.

Systeme zur Ermittlung des Geschlechtes eines Embryos sind bereits seit geraumer Zeit im Einsatz bei der kommerziellen Aufzucht von Nutztieren wie beispielsweise Küken. Da es dabei wünschenswert ist, die Embryonen, insbesondere die weiblichen Embryonen, nicht zu beschädigen, um eine weitere Aufzucht nicht zu gefährden sowie Verbrauchsmaterialien und den mechanischen Aufwand zu minimieren, sind dabei insbesondere nicht invasive Vorrichtungen und Methoden von Vorteil.

Den meisten dieser Vorrichtungen ist gemein, dass sie über das sogenannte "candling" das Geschlecht des Embryos bestimmen. Dabei wird das Ei durchleuchtet. Anhand der wieder aus dem Ei austretenden Strahlung werden Informationen über den Zustand des Eis beziehungsweise des Embryos gesammelt.

Aufgrund der großen biologischen Varianz in den Eigenschaften des Eis, wie Größe, Form, Farbe und Schalendicke, und der damit verbundenen großen Breite möglicher Messergebnisse, ist es allerdings schwierig, verlässliche quantitative Aussagen zu treffen. Insbesondere in den frühen Entwicklungsstadien des Embryos, in denen die gesuchten Signale noch recht schwach ausgeprägt sind, erschweren diese Faktoren die Geschlechtsbestimmung daher erheblich.

Eine frühphasige verlässliche Erkennung des Geschlechtes eines Embryos ist erforderlich. Insbesondere aufgrund ethischer Bedenken und den resultierenden gesetzlichen Vorgaben ist es dabei wesentlich, eine Geschlechtserkennung möglichst früh, insbesondere vor der Entwicklung eines Schmerzempfindens zu ermöglichen (Bruttag 7), um sowohl das Tierleid in der der Legehennenzucht zu reduzieren, als auch Brütereien eine kostengünstige Alternative für die ressourcen- und kostenintensive Aufzucht der männlichen Legehennenküken anzubieten, die aufgrund ihrer andersartigen Fleischkonsistenz nur als Nischenprodukt verwendet werden können und keine Abnehmer finden.

Da in der kommerziellen Aufzucht von Eiern zudem große Mengen an zu bestimmenden Eiern anfallen, ist es ferner schwierig, Messsysteme bereitzustellen, welche in der Lage sind, einen entsprechend hohen Durchsatz zu ermöglichen, ohne dabei durch große Mengen an benötigter Sensorik übermäßige Kosten zu generieren.

Eine weitere Schwierigkeit besteht in der häufig angetroffenen Anforderung, Messsysteme bereitzustellen, die mit bereits vorhandenen Inkubatoren und Eier-Trays kompatibel sind, welche zur Lagerung und Inkubation der Eier verwendet werden, um Neuanschaffungen möglichst zu vermeiden.

Im Lichte der vorstehenden Ausführungen besteht die Aufgabe der vorliegenden Erfindung darin, ein System und ein Verfahren bereitzustellen, welche es erlauben, große Mengen an Eiern zur Bestimmung ihres Geschlechts zu untersuchen, wobei schon früh im Brutzyklus eine hohe Genauigkeit erreicht wird. Ferner soll es möglich sein, ein hohes Maß von Integrierbarkeit in bereits bestehende Aufzuchtsysteme zu realisieren.

Diese Aufgabe wird gelöst durch ein Messsystem mit den Merkmalen von Anspruch. Die Unteransprüche geben bevorzugte Weitergestaltungen an.

Die Aufgabe wird insbesondere gelöst durch ein Messsystem zur nicht-invasiven, vorzugsweise automatisierten, Geschlechtserkennung von Embryos in einem Ei in der früh-embryonalen Entwicklung, insbesondere vor dem siebten Bruttag, insbesondere während der Inkubation, insbesondere in einem Inkubator mit mindestens einem Eier-Tray zur Aufnahme einer Vielzahl von Eiern und mindestens einem Eier-Trolley zur Halterung mindestens eines Eier-Trays, aufweisend:
- mindestens eine Bestrahlungseinheit zur Bestrahlung eines Eis mit elektromagnetischer Strahlung;
- mindestens eine Sensoreinheit zur Erfassung von durch das Ei transmittierter elektromagnetischer Strahlung;
- eine Auswertungseinheit mit
   ∘ mindestens einem Spektrometer, das vorzugsweise mit der Sensoreinheit verbunden ist, und das dazu ausgebildet ist, die durch das Ei transmittierte Strahlung zu empfangen und ein Spektrum der durch das Ei transmittierten Strahlung zu erzeugen; und
   ∘ einer Datenverarbeitungseinheit, die dazu ausgebildet ist, von dem Spektrometer erzeugte Spektren zu empfangen und als Referenzspektrum oder Messspektrum abzuspeichern;
- eine Identifikationseinheit zur Erzeugung von Identifikationsdaten, mittels derer die an einem Ei erzeugten Spektren dem Ei eindeutig zuordenbar sind;
- eine Klassifikationseinheit;
wobei die Datenverarbeitungseinheit dazu ausgebildet ist, von dem Spektrometer erzeugte Spektren und zugehörige Identifikationsdaten zu speichern, und wobei die Klassifikationseinheit dazu ausgebildet ist, das Geschlecht des Embryos auf Grundlage mindestens eines Referenzspektrums und mindestens eines Messspektrums bestimmt.

Ein wesentlicher Gedanke der Erfindung besteht darin, dass einem einzelnen Ei ein individualisiertes Referenzspektrum zuordenbar ist, welches dazu verwendet werden kann, die durch die biologische Varianz verursachten Störfaktoren aus einer Messung zu berücksichtigen, und somit eine präzisere Bestimmung des Geschlechts des Embryos zu einem frühen Zeitpunkt zu ermöglichen.

Dabei werden aus durch das Ei transmittierter (elektromagnetischer) Strahlung Transmissions-Daten erzeugt, die zu einem Spektrum zusammengestellt werden. Basierend auf dem ermittelten Spektrum, dass sich hierbei aus einer einzelnen oder mehreren Messungen der durch das Ei transmittierten Strahlung (Transmissions-Daten) zusammensetzen kann, können Rückschlüsse auf spektrale Absorptionsbereiche im Ei gezogen werden. Die von der Datenverarbeitungseinheit erzeugten Informationen werden dabei an die Klassifikationseinheit weitergeleitet, welche die letztendliche Einteilung vornimmt.

Die Komponenten der Auswertungseinheit können durch kommunikativ miteinander verbundene, separate Einheiten bereitgestellt werden. Ebenso können mehrere oder alle Einheiten der Auswertungseinheit als gemeinsame bauliche Einheit vorgesehen werden. Die Datenverarbeitungs-, Identifikations- und Klassifikationseinheit können durch Mikroprozessoren oder Softwarekomponenten gebildet sein, die auf einer Rechenvorrichtung gespeichert sind und dort ausführbar sind, um die anspruchsgemäßen Funktionen bereitzustellen.

Gemäß einer bevorzugten Ausführungsform weist das Messsystem ein optisches Entkopplungselement zur optischen Entkopplung der Bestrahlungseinheit und der Sensoreinheit auf, das vorzugsweise dazu ausgebildet ist, während einer Messung an dem Ei anzuliegen.

Ein wesentlicher Störfaktor bei der Messung eines Spektrums, insbesondere eines Transmissionsspektrums, ist Streulicht, welches in den Sensor gelangt, ohne vorher das Ei durchlaufen zu haben, da dieses von hoher Intensität ist, ohne jedoch irgendwelche relevante spektrale Information zu tragen. Um dies zu verhindern, wird ein optisches Entkopplungselement genutzt, welches verhindert, dass Licht direkt von der Bestrahlungseinheit in den Sensor gelangen kann. Ein solches Entkopplungselement kann dabei die Form einer Blende oder einer Bürste haben, welche möglichst dicht an der Schale des Eis anliegt, um wenige mögliche Spalten für den Austritt oder Eintritt von Streulicht offen zu lassen. Es versteht sich dabei von selbst, dass bei der Verwendung von Strahlung außerhalb des sichtbaren Bereiches eine entsprechend abdichtende Blende verwendet werden kann.

Das Entkopplungselement kann derart ausgebildet und angeordnet sein, dass es die mindestens eine Bestrahlungseinheit gegenüber der Umgebung abschirmt. Insbesondere kann das Entkopplungselement derart angeordnet und ausgebildet sein, dass ein Ei derart an dem Entkopplungselement anordenbar ist, dass von der Bestrahlungseinheit abgegebene Strahlung im Wesentlichen vollständig an das Ei geleitet wird. Ebenso kann das Entkopplungselement derart ausgebildet und angeordnet sein, dass es die mindestens eine Sensoreinheit gegenüber der Umgebung abschirmt. Insbesondere kann das das Entkopplungselement derart angeordnet und ausgebildet sein, dass ein Ei derart an dem Entkopplungselement anordenbar ist, dass die Sensoreinheit gegenüber der Umgebung des Eis abgeschirmt ist.

Vorzugsweise weist das Messsystem Mittel zur Bestimmung eines Winkels zwischen einer Referenzachse des Eis und einer Referenzachse des Messsystems auf. Als Referenzachse des Messsystems kann eine ortsfeste Referenzachse, beispielsweise die Vertikale (also eine Richtung parallel zur Richtung der Schwerkraft) dienen. Als Referenzachse des Eis kann eine Achse gewählt werden, die durch die beiden Spitzen des Eis verläuft, also eine Achse, zu der die Schale des Eis im Wesentlichen rotationssymmetrisch ist.

Nachdem der Embryo sich in den ersten Entwicklungstagen bei jeder Lage des Eis so ausrichten wird, dass er oben aufschwimmt, ist die Kenntnis eines Kippwinkels des Eis bzw. der optischen Messachse relativ zu einer Referenzachse des Messsystems förderlich, um sicherzustellen, wo sich der Embryo in Bezug auf das Sichtfeld der Sensoreinheit befindet. Ferner führt die Messung unter verschiedenen Kippwinkeln zu einer Erhöhung der Varianz der ermittelten Spektren. Die Kenntnis des Kippwinkels des Eis kann zusätzlich genutzt werden, um eine spätere Vergleichbarkeit der Referenzspektren mit den Messspektren zu verbessern, indem beispielsweise der Embryo durch die Kombination verschiedener Messungen bei verschiedenen Kippstellungen herausgerechnet wird.

Hierbei ist es insbesondere bevorzugt, dass die Referenzachse des Messsystems durch eine ortsfeste Achse, beispielsweise die Vertikale gebildet, ist, die auch bei einer Bewegung des Messsystems unveränderlich ist. Ist das Messsystem derart ausgebildet, dass die Position und Orientierung des Eis relativ zur Strahlungsquelle und der Sensoreinheit festlegbar sind, führt eine Verkippung des Eis gemeinsam mit der Strahlungsquelle und der Sensoreinheit zu keiner Änderung der relativen Position und Orientierung des Eis bezüglich der Strahlungsquelle und der Sensoreinheit. Dennoch ändert sich dabei die Position des Embryos relativ zu Strahlungsquelle und Sensoreinheit.

Die Verkippung ist auch in diesem Fall bestimmbar, wenn als Referenzachse des Messsystems eine ortsfeste Achse wie beispielsweise die Vertikale gewählt wird. Als Mittel zur Bestimmung des Winkels zwischen der Referenzachse des Eis und einer ortsfesten Referenzachse des Messsystems wie der Vertikalen kann beispielsweise ein Gyroskop verwendet werden, das vorzugsweise starr mit einer Komponente des Messsystems verbunden ist.

Nachdem der Embryo in der Entwicklung zwar aufschwimmt, aber im Laufe der Entwicklung häufig dezentral an der Eierschale anwächst und daher selten genau mittig positioniert ist, sodass er auf der Symmetrieachse des Eis liegt, ist es bevorzugt, die Bestrahlung des Eis von verschiedenen Richtungen vornehmen zu können.

Hierbei ist es bevorzugt, dass die Bestrahlungseinheit eine Vielzahl von Strahlungsquellen aufweist. Besonders bevorzugt ist die Bestrahlungseinheit durch eine Ringleuchte, besonders bevorzugt durch eine Ring-LED, gebildet, bei der eine Vielzahl von Strahlungsquellen, vorzugsweise LEDs, auf einem Ring angeordnet ist. Vorzugsweise ist der Durchmesser der Ringleuchte so gewählt, dass er kleiner ist als der Durchmesser eines Eis an seiner dicksten Stelle senkrecht zur Symmetrieachse des Eis. Eine solche, als Ringleuchte ausgebildete, Strahlungsquelle kann an dem stumpfen Ende des Eis angeordnet werden und erlaubt eine Bestrahlung des Eis aus verschiedenen Positionen bzw. Richtungen, die radial um die Symmetrieachse des Eis angeordnet sind.

Vorzugsweise weist die Ringleuchte genau oder mindestens 4 Strahlungsquellen (beispielsweise LEDs), weiter vorzugsweise genau oder mindestens 8 Strahlungsquellen auf, die vorzugsweise gleichmäßig entlang des Umfangs der Ringleuchte verteilt angeordnet sind. Hilfreich dabei kann die Verwendung von Strahlbegrenzern um die einzelnen Strahlungsquellen sein, um die unterschiedlichen bestrahlten Bereiche des Eis klar voneinander abzugrenzen. Vorzugsweise sind die Strahlungsquellen der Beleuchtungseinheit unabhängig voneinander ansteuerbar ausgebildet, um eine selektive Bestrahlung des Eis aus verschiedenen Richtungen zu ermöglichen.

Gemäß einer weiteren Ausführungsform weist die Bestrahlungseinheit eine Vielzahl von optische Lichtführungen auf, die dazu ausgebildet sind, entlang eines Rings an einem Ende des Eis angeordnet zu werden, und die unabhängig voneinander ansteuerbar sind, um die von der Bestrahlungseinheit emittierte Strahlung aus verschiedenen Richtungen auf das Ei zu leiten.

Die Sensoreinheit bleibt entweder statisch an einem der beiden Pole positioniert oder wird auf der gegenüberliegenden Seite der aktiven Beleuchtungsrichtung positioniert. In jedem Fall ist es bevorzugt, dass das optische Entkopplungselements lichtdicht am Ei anordenbar ist.

Vorzugsweise ist das Messsystem dazu ausgebildet, eine Vielzahl von Messungen durchzuführen, bei denen das Ei jeweils aus unterschiedlichen Richtungen beleuchtet wird, indem nacheinander einzelne Strahlungsquellen aktiviert werden oder die von der Bestrahlungseinheit emittierte Strahlung nacheinander durch verschiedene optische Lichtführungen an das Ei geleitet wird.

Durch den Vergleich der nacheinander ausgeführten Messungen untereinander kann eine Messung identifiziert werden, in der der Embryo am stärksten bestrahlt wird, also eine Messung mit einem relativ höchsten und/oder stärksten Nutzsignal. Ein Teil der übrigen Messungen oder alle übrigen Messungen, in denen das Nutzsignal kleiner und/oder schwächer war, können mit der Messung mit dem relativ höchsten und/oder stärksten Nutzsignal verrechnet werden (beispielsweise mittels Division oder Subtraktion), um die Qualität der Messung und des daraus generierten Messspektrums zu verbessern.

Gemäß einer bevorzugten Ausführungsform sind die Bestrahlungseinheit und die Sensoreinheit derart angeordnet und konfiguriert, dass sie bei jeder Messung relativ zu zwei Bezugspunkten des Eis, wie beispielsweise zum Schwerpunkt der Schale des Eis oder den zwei Polen bzw. Spitzen des Eis, dieselbe Ausrichtung zueinander haben.

Es ist vorteilhaft, sicherzustellen, dass sich die Elemente des Messsystems, insbesondere die Bestrahlungseinheit und die Sensoreinheit, immer im Wesentlichen in der gleichen relativen Anordnung und Ausrichtung zueinander und zum Ei befinden, da hierdurch die Varianz zwischen den Messungen erheblich reduziert werden kann. In Verbindung mit der Bestimmung des Winkels einer Ei-Achse können ferner gezielt Messungen aus verschiedenen Winkeln durchgeführt werden.

Eine weitere Ausführungsform der vorliegenden Erfindung beinhaltet eine Trolley-Transportvorrichtung zum Transport eines Eier-Trolleys mit mindestens einem Eier-Tray zu mindestens einer Bestrahlungseinheit, die vorzugsweise innerhalb des Inkubators angeordnet ist, weiter vorzugsweise zwischen verschiedenen Inkubatoren und/oder eine Trolley-Positionierungsvorrichtung, welche dazu geeignet ist, eine Position des Trolleys innerhalb des Inkubators eindeutig festzulegen. In diesem Fall ist es bevorzugt, dass die mindestens eine Bestrahlungseinheit und die mindestens eine Sensoreinheit an einer Messsäule angebracht sind, die ortsfest in dem Inkubator installierbar ist. Wie im Folgenden noch näher beschrieben wird, können die mindestens eine Bestrahlungseinheit und die mindestens eine Sensoreinheit dabei fest oder beweglich an der Messsäule angebracht sein.

Nachdem in der kommerziellen Eiaufzucht in der Regel sogenannte Eier-Trolleys verwendet werden, um eine große Menge von Eier-Trays aufzunehmen und innerhalb eines Inkubators zu halten, ist es vorteilhaft, wenn ein vollautomatisierter oder zumindest teilautomatisierter Transport der Trolleys zu der Bestrahlungs- und/oder Sensoreinheit erfolgen kann. Dies bietet den Vorteil, dass das Messsystem in bereits vorhandenen Inkubatoren eingesetzt werden kann, ohne dass das bereits vorhandene Equipment ausgetauscht werden müsste. Hierbei bieten sich insbesondere Lösungen an, bei welchen die Transportvorrichtung bereits die Position aller Trolleys innerhalb des Inkubators kennt, um diese dann zielgerichtet anzufahren. Dazu kann die Trolley-Transportvorrichtung als Roboter ausgebildet sein, der eine Steuereinheit aufweist, die dazu ausgebildet ist, die Trolley-Transportvorrichtung an vorab festlegbare Positionen innerhalb des Inkubators zu steuern, um Eier-Trolleys von vorab festlegbaren Positionen zu der Bestrahlungs- und/oder Sensoreinheit zur Messung zu transportieren.

Darüber hinaus ist eine genaue Kenntnis der Position der Trolleys oder die Trolley-Positionierungsvorrichtung auch hilfreich, um die Identifizierung einzelner Trolleys, Eier-Trays oder Eier zu Zwecken der Messung, Weiterbeobachtung oder Aussortierung zu ermöglichen. Vorzugsweise steht die Trolley-Transportvorrichtung in kommunikativer Verbindung mit der Auswertungseinheit und/oder der Identifikationseinheit und ist dazu ausgebildet, Trolley-Identifikationsdaten an die Auswertungseinheit und/oder der Identifikationseinheit zu übertragen, die bei der Erzeugung der Identifikationsdaten berücksichtigt werden.

Weiter wird die Aufgabe der Erfindung gelöst durch ein Messsystem aufweisend Transportmittel zum Transport der Bestrahlungseinheit und der Sensoreinheit zu einem Ei, vorzugsweise innerhalb des Inkubators, weiter vorzugsweise zwischen verschiedenen Inkubatoren.

Ergänzend oder alternativ zum vorstehend beschriebenen, erfindungsgemäßen Konzept, die Eier-Trolleys zu der Bestrahlungs- und/oder Sensoreinheit zu transportieren, um Messungen an Eiern durchzuführen, ist es ebenfalls im Sinne der vorliegenden Erfindung, das Messsystem so zu konfigurieren, dass die Bestrahlungseinheit und die Sensoreinheit zu einem (designierten) Eier-Tray transportierbar sind. Dies ist insbesondere deshalb von Vorteil, weil die Eier bei dieser Ausführungsform durch die Messungen weniger in ihrer Inkubation gestört und dadurch einem geringeren Risiko ausgesetzt werden, im Entwicklungsprozess beschädigt zu werden oder abzusterben.

Gemäß einem Aspekt der vorliegenden Erfindung wird ein Messsystem bereitgestellt, bei dem die Trolley-Transportvorrichtung Mittel aufweist, die dazu ausgebildet sind, eine Kippstellung mindestens eines Eis einzustellen.

Um ein möglichst einfaches und mit bereits bestehenden Trolley-Elementen kompatibles Einstellen der Kippstellung mindestens eines Eis bzw. des Winkels der Referenzachse des Eis zu ermöglichen, ist es vorteilhaft, die Trolley-Transportvorrichtung mit ergänzenden oder interagierenden Komponenten auszurüsten, um eine hohe Kompatibilität und somit Kostenersparnis zu erreichen. Hierfür ist es bevorzugt, dass der Trolley-Transportvorrichtung Mittel zur Einstellung des Kippwinkels der Eier-Trays in einem Eier-Trolley aufweist. Die Mittel zur Einstellung des Kippwinkels der Eier-Trays in einem Eier-Trolley sind vorzugsweise dazu ausgebildet, mit einer Kippvorrichtung des Eier-Trolleys in Eingriff zu kommen und/oder in Wechselwirkung zu treten, um den Kippwinkel der Eier-Trays im Eier-Trolley einzustellen. So kann zum Beispiel die Trolley-Transportvorrichtung dazu ausgebildet sein, den Eier-Trolley als Ganzes mit darin befindlichen Eier-Trays und Eiern zu kippen, oder in eine vorhandene Kippvorrichtung des Eier-Trolleys zum Kippen der Eier-Trays einzugreifen.

Eine weitere Fortbildung der vorliegenden Erfindung umfasst ein Messsystem aufweisend einen Messarm, welcher die (mindestens eine) Bestrahlungseinheit und die (mindestens eine) Sensoreinheit derart aufnimmt, dass mindestens ein Ei zur Messung an der (mindestens einen) Bestrahlungseinheit und/oder der (mindestens einen) Sensoreinheit positionierbar ist.

Eine gemeinsame Unterbringung der Bestrahlungs- und Sensoreinheit in einem Messarm birgt den Vorteil, dass dies die relative Positionierung der beiden Einheiten zueinander und relativ zum Ei erleichtert. Weiterhin wird dadurch eine Anpassung der Messvorrichtung an einen Winkel eines Eier-Trays deutlich vereinfacht, da lediglich der Winkel und die Position des Messarms angepasst werden muss.

Gemäß einem weiteren Aspekt der vorliegenden Erfindung enthält das Messsystem einen ersten Messarm, welcher die (mindestens eine) Bestrahlungseinheit aufnimmt, und einen zweiten Messarm, welcher die (mindestens eine) Sensoreinheit aufnimmt, wobei der erste Messarm und der zweite Messarm derart angeordnet und ausgebildet sind, dass mindestens ein Ei zur Messung zwischen der (mindestens einen) Bestrahlungseinheit und der (mindestens einen) Sensoreinheit positionierbar ist.

Diese Konstruktion ermöglicht eine Anordnung der Bestrahlungseinheit relativ zur Sensoreinheit an gegenüberliegenden Enden des Eis. Dies birgt den Vorteil, dass das Messsystem dazu eingerichtet werden kann, die Eier in Durchleuchtung zu vermessen. Dadurch kann im Vergleich zu einer Detektion von zurück oder zur Seite gestreuter Strahlung die Luminosität erhöht werden.

In einem weiteren Aspekt der vorliegenden Erfindung weist das Messsystem folgendes auf: eine Messsäule, an der der erste Messarm und der zweite Messarm beweglich gelagert sind, wobei der erste Messarm einen ersten Bewegungsmechanismus zur Einstellung einer Vertikalposition des ersten Messarms aufweist, und wobei der zweite Messarm einen zweiten Bewegungsmechanismus zur Einstellung einer Vertikalposition des zweiten Messarms aufweist.

Eine bewegliche Anordnung der Messarme ermöglicht dabei eine Messung an einer Vielzahl an Eier-Trays, ohne für jede Ebene einen eigenen Messarm zu benötigen. Dadurch kann die Anzahl der insgesamt benötigten Bestrahlungs- und Sensoreinheiten klein gehalten werden. Dies ermöglicht unter anderem eine einfache Integration in bereits bestehende Brutvorrichtungen.

Eine weitere mögliche Ausführungsform weist ein erfindungsgemäßes Messsystem auf, wobei der erste Bewegungsmechanismus eine erste Horizontal-Linearführung und eine zweite Horizontal-Linearführung zur Einstellung einer Horizontalposition des ersten Messarms aufweist, und wobei der zweite Bewegungsmechanismus eine dritte Horizontal-Linearführung und eine vierte Horizontal-Linearführung zur Einstellung einer Horizontalposition des zweiten Messarms aufweist.

Durch eine derartige Linearführung lassen sich die Bestrahlungseinheit und die Sensoreinheit relativ zueinander frei bewegen und positionieren. Dadurch wird eine einfache Möglichkeit zum Wechsel zwischen verschieden Eier-Trays geschaffen, was sowohl den Messdurchsatz als auch die Kompatibilität erhöht und damit Kosten spart.

Weiterhin besteht eine Ausgestaltungsmöglichkeit der vorliegenden Erfindung in einem Messaufsatz, welcher sowohl die Bestrahlungseinheit als auch die Sensoreinheit aufnimmt, so dass sowohl die Bestrahlungseinheit als auch die Sensoreinheit, insbesondere für eine Messung, entweder über oder unter dem Eier-Tray anordenbar ist. Der Messaufsatz ist dazu ausgestaltet, während der Messung an das Eier-Tray angesetzt zu werden. Hierzu kann der Messaufsatz einen durch Messaufsatz-Adapter aufweisen, der eine festgelegte Positionierung des Messaufsatzes relativ zu einem Eier-Tray ermöglicht.

Ein derartiger Messaufsatz birgt den Vorteil, dass er ohne große Komplikationen auf bereits in Verwendung befindliche Eier-Trays aufgesetzt werden kann. Dies ist insbesondere deshalb von Vorteil, da dadurch hohe Umrüstungskosten vermieden werden. Zudem kann der Aufsatz auf einfache Weise sowohl manuell, als auch von einer automatisierten Transportvorrichtung zwischen verschiedenen Eier-Trays, Eier-Trolleys und auch Inkubatoren hin und her bewegt werden. Zudem passt sich ein derartiger Aufsatz von selbst an einen veränderbaren Winkel des Eier-Trays an, ohne eine neue Ausrichtung zu erfordern.

In einer bevorzugten Ausführungsform der Erfindung umfasst das Messsystem mindestens eine Belüftungsöffnung, die in dem Messaufsatz, dem ersten Messarm und/oder dem zweiten Messarm ausgebildet ist und dazu ausgestaltet ist, während einer Messung eine Belüftung des Eis sicherzustellen.

Für optimale Brutbedingungen der Eier ist es notwendig, einen möglichst großflächigen Teil der Eier in ständigem Wärmeaustausch mit der Umgebungsluft des Inkubators zu halten. Ein erfindungsgemäßer Belüftungskanal stellt somit sicher, dass auch während der Messung ein stetiger Strom von Inkubator Luft in Wärmeaustausch mit der Oberfläche des Eis steht. Als Lüftungskanal können dabei alle Öffnungen verstanden werden, welche es der Inkubatorluft während des Messvorgangs erlauben, in Kontakt mit dem Ei zu gelangen. Insbesondere kann die Belüftungsöffnung durch eine poröse oder wabenförmige Struktur gebildet werden, um einen möglichst großen Luftaustausch bereitzustellen.

In einer weiteren bevorzugten Ausgestaltung der vorliegenden Erfindung ist die Auswertungseinheit dazu ausgebildet, für jedes Ei ein Geschlechtslabel und eine zugehörige Konfidenz auszugeben. Diese kann auch an die Klassifikationseinheit ausgegeben werden.

Da die Identifikation des Geschlechtes eines Embryos gerade in der frühen Brutphase eventuell nicht absolut zuverlässig erfolgen kann, ist es von großem Vorteil, die Einteilung von Eiern in die beiden Geschlechter mit einer Konfidenz zu versehen. Die Konfidenz kann dann beispielsweise als Selektionskriterium genutzt werden. So kann beispielsweise der Erwartungswert einer bestimmten Anzahl von männlichen oder weiblichen Embryonen maximiert oder minimiert werden, oder eine vorbestimmte Wahrscheinlichkeit für eine vorbestimmte Mindestzahl an weiblichen Embryonen ermittelt werden. Die Konfidenz kann durch verschiedene algorithmische Verfahren basierend auf Mess- und Referenzspektren im Klassifikationsprozess erzeugt werden. Sie stellt dar, wie eindeutig die Einteilung in das Geschlecht zu beurteilen ist und ist demnach ein Maß für die Unsicherheit.

In einer weiteren Ausführungsform kann das Messsystem einen (extern angebundenen) Datenspeicher aufweisen, insbesondere einen Cloud-Speicher, der dazu konfiguriert ist, externe Parameter wie Sterberate der Embryos oder gewünschte Outputmenge, Mess- und Referenzspektren, und/oder Ergebnisse der Auswertung des Messsystems zu speichern und an die Auswertungseinheit bzw. Klassifikationseinheit auszugeben.

Dies hat den vorteilhaften Effekt, dass so auf einfache Weise Selektionsentscheidungen getroffen werden können, welche insbesondere auch die Messergebnisse anderer Trolleys oder Inkubatoren miteinbeziehen um eine Gesamtoutputmenge sinnvoll zu steuern. Die externen Parameter sind durch einen Betreiber des Inkubators vorgebbar und anpassbar.

Gemäß einer weiteren Ausführungsform der vorliegenden Erfindung umfasst das Messsystem Befestigungsmittel, welche dazu ausgebildet sind, eine Änderung der Ausrichtung des Eis in Bezug zum Eier-Tray zu verhindern.

Nachdem die Winkelstellung des Eis durch ein Aufschwimmen des Embryos einen großen Einfluss auf die gemessenen Spektren hat, lässt sich über eine Fixierung dieses Winkels eine höhere Reproduzierbarkeit und Genauigkeit bei allen Messungen, sowie beim Voreinstellen oder Ändern des Winkels erzielen. Damit wird die Genauigkeit und Verlässlichkeit der Geschlechtsbestimmung weiter verbessert. Dabei können die Befestigungsmittel sowohl Bestandteil eines der Messarme, bzw. des Messaufsatzes oder Messaufsatz-Adapter, sein, als auch Bestandteil eigens zu diesem Zweck als Teil des Messsystems bereitgestellter Spezial-Eier-Trays.

Eine weitere erfindungsgemäße Ausgestaltung umfasst das obige Messsystem, wobei die Auswertungseinheit eine Klassifikationseinheit aufweist, welche anhand der Daten der Messeinheit und/oder aus dem extern angebundenen Speicher eine Klassifikation eines Eis nach Geschlecht und/oder Gesundheitszustand vornimmt, wobei die Klassifikationseinheit vorzugsweise räumlich vom Rest der Auswertungseinheit getrennt ist. Insbesondere kann die Klassifikationseinheit durch eine Softwarekomponente auf einem externen Server, vorzugsweise auf einem Cloud-Server, gebildet sein.

Eine Trennung der Auswertungs- und Klassifikationseinheit kann es erleichtern, eine Steuerung des Outputs mehrerer Trolleys oder Inkubatoren zu übernehmen. In diesem Fall ist nur eine Vorbearbeitung der gemessenen Daten vor Ort nötig, während die tatsächlichen Selektionsentscheidungen aufgrund größerer Datenmengen räumlich und/oder zeitlich getrennt erfolgt.

Die Aufgabe der Erfindung wird ferner gelöst durch ein Verfahren zur nicht-invasiven Geschlechtserkennung eines Embryos in einem Ei in der früh-embryonalen Entwicklung, insbesondere vor dem siebten Bruttag, insbesondere während der Inkubation, aufweisend die folgenden Schritte:
- Erzeugung mindestens eines Referenzspektrums vor und/oder zu Beginn der Inkubation mittels Bestrahlung des Eis mit elektromagnetischer Strahlung und Detektion von durch das Ei hindurchgetretener Strahlung, Erzeugung von Identifikationsdaten zur eindeutigen Identifizierung des Eis und Abspeichern des Referenzspektrums zusammen mit Identifikationsdaten des Eis;
- Erzeugung mindestens eines Messspektrums während der Inkubation mittels Bestrahlung des Eis mit elektromagnetischer Strahlung und Detektion von durch das Ei hindurchgetretener Strahlung;
- Auswertung des Messspektrums unter Verwendung des zum Ei gehörigen gespeicherten Referenzspektrums zur Bestimmung des Geschlechts des Embryos.

Bei dem erfindungsgemäßen Verfahren kann das Referenzspektrum und/oder das Messspektrum aus einer Vielzahl an Einzelmessungen erzeugt werden, vorzugsweise aus 10 Messungen oder mehr, weiter vorzugsweise aus 30 Messungen oder mehr.

Durch eine Kombination aus verschiedenen Messungen erhöht sich die Zuverlässigkeit eines Spektrums aufgrund statistischer Unterdrückung von randomisierten Störfaktoren.

Eine weitere Fortbildung des erfindungsgemäßen Verfahrens besteht darin, dass für die Erzeugung eines Mess- oder Referenzspektrums eine Bestrahlungsdauer von weniger als 60 µs, vorzugsweise weniger als 40 µs, weiter vorzugsweise von weniger als 20 µs genutzt wird.

Dies ist einerseits von Vorteil, da bei einer möglichen Vielzahl von Messungen an einer sehr großen Menge von Eiern so eine große Zeitersparnis erzeugt wird. Andererseits minimiert eine kurze Bestrahlungsdauer die gesundheitlichen Risiken für den Embryo.

Gemäß einem weiteren Gedanken der Erfindung wird ein Winkel zwischen einer Längsachse des Eis und einer Referenzachse des Messsystems bestimmt. Als Referenzachse des Messsystems kann wiederum eine ortsfeste Achse wie beispielsweise die Vertikale dienen. Als Referenzachse des Eis kann eine Achse gewählt werden, die durch die beiden Spitzen des Eis verläuft, also eine Achse, zu der die Schale des Eis im Wesentlichen rotationssymmetrisch ist.

Nachdem der Embryo in der frühen Brutphase relativ zur Richtung der Schwerkraft obenauf schwimmt, ermöglicht eine Bestimmung und/oder Anpassung des Kippwinkels eines Eis eine wunschgemäße Positionierung des Embryos relativ zur Ei-Schale. Ferner können durch die Bestimmung des Winkels zwischen der Referenzachse des Eis und der Messachse des Messsystems, die durch variierende Kippwinkel des Messsystems samt Ei bedingten Varianzen in den Messungen erheblich reduziert werden. Die Kenntnis des Kippwinkels des Messsystems samt Ei kann zusätzlich genutzt werden, um eine spätere Vergleichbarkeit der Referenzspektren mit den Messspektren zu verbessern.

Gemäß einer weiteren Ausgestaltungsmöglichkeit der vorliegenden Erfindung wird nach einer erfolgten Messung der Winkel des Eis (also der Winkel der Längsachse des Eis relativ zu einer Referenzachse des Messsystems) verändert und eine weitere Messung mit dem veränderten Winkel des Eis wird durchgeführt, nachdem das Ei einen Gleichgewichtszustand erreicht hat. Zur Erreichung des Gleichgewichtszustands kann nach einer Veränderung des Winkels des Eis eine vorbestimmte Wartezeit, z.B. mindestens 5 Sekunden oder mindestens 10 Sekunden, abgewartet werden, bevor eine weitere Messung mit dem veränderten Winkel des Eis durchgeführt wird.

Besonders zu Zwecken einer Referenzmessung kann dies von Vorteil sein, um beispielsweise den Embryo aus dem Bildbereich der Sensoreinheit heraus zu kippen, was einen größeren Kontrast zu späteren Messungen mit Embryo ermöglicht. Dadurch erhöht sich die Genauigkeit der Geschlechtsbestimmung gerade in den Frühphasen der embryonalen Entwicklung.

Es ist weiter bevorzugt, dass zur Erzeugung eines Referenzspektrums und/oder eines Messspektrums eine Vielzahl von Messungen durchgeführt wird, bei denen das das Ei aus (mindestens) 4 unterschiedlichen Richtungen, vorzugsweise (mindestens) 6 unterschiedlichen Richtungen, weiter vorzugsweise aus 8 oder mehr unterschiedlichen Richtungen bestrahlt wird. Vorzugsweise wird für jede unterschiedliche Richtung der Bestrahlung eine Messung durchgeführt, um verschiedene Spektren für unterschiedliche Bestrahlungsrichtungen zu erhalten, die zu einem Referenzspektrum bzw. Messspektrum verarbeitet werden können.

Dies kann beispielsweise durch die Verwendung einer Bestrahlungseinheit mit einer Vielzahl von Strahlungsquellen erreicht werden, besonders bevorzugt durch die Verwendung einer Ringleuchte, bei der eine Vielzahl von Strahlungsquellen in einer ringförmigen Bestrahlungseinheit entlang des Umfangs des Rings angeordnet sind und unabhängig voneinander ansteuerbar bzw. aktivierbar sind. Es ist auch denkbar, die Ausrichtung der Bestrahlungseinheit relativ zum Ei zu verändern.

Weiterhin umfasst die vorliegende Erfindung ein bevorzugtes Verfahren, wobei nacheinander Messungen bei Bestrahlung des Eis aus unterschiedlichen Richtungen werden und die jeweilige Stärke eines Nutzsignals in den Messungen ermittelt wird. Vorzugsweise werden auf Grundlage der jeweiligen Stärke des Nutzsignals ein oder mehrere Messungen mit dem stärksten und/oder höchsten Nutzsignal identifiziert.

Dies ist besonders von Vorteil, da der Embryo nicht immer zentral im Ei obenauf schwimmt, sondern häufig einen seitlichen Versatz zur mittleren Symmetrieachse des Eis hat und im späteren Entwicklungsstadium dezentral anwächst. So kann die Signalqualität (und damit die Verlässlichkeit der Geschlechtsbestimmung) durch eine Auswahl einer Messung mit dem vergleichsweise stärksten und/oder höchsten Nutzsignal verbessert werden.

Weiterhin ist es bevorzugt, dass die Messung mit dem stärksten bzw. höchsten Nutzsignal mit einer oder mehrerer Messungen mit schwächerem bzw. niedrigerem Nutzsignal verrechnet wird, um ein (optimiertes) Referenz- bzw. Messspektrum zu erhalten. Die Verrechnung kann vorzugsweise das Dividieren, Subtrahieren und/oder Mitteln verschiedener Messungen umfassen.

Somit können beispielsweise beliebige Teilmengen der Vielzahl an Messungen mit unterschiedlichen Beleuchtungsrichtungen miteinander verrechnet oder verglichen werden. Beispielsweise können alle Messungen außer der mit dem stärksten Nutzsignal gemittelt, und das Ergebnis mit der Messung mit dem stärksten Nutzsignal verrechnet werden. Die Verrechnung kann derart erfolgen, dass die Messung mit dem stärksten Nutzsignal durch die (gemittelte) Messung mit schwächerem Nutzsignal dividiert wird, oder indem die (gemittelte) Messung mit schwächerem Nutzsignal von der Messung mit dem stärksten Nutzsignal subtrahiert wird. Die Messung mit dem stärksten Nutzsignal kann beispielsweise anhand der absoluten Absorption in einem bestimmten Spektralbereich ermittelt werden. Dies steigert die Signalzuverlässigkeit und die Reliabilität der Messungen.

Weiterhin umfasst die vorliegende Erfindung ein Verfahren, bei dem anhand eines gespeicherten Referenzspektrums eine Normalisierung eines Messspektrums durchgeführt wird, wobei das Referenzspektrum vorzugsweise vor der Inkubation, weiter vorzugsweise innerhalb des Inkubators, weiter vorzugsweise vor Erreichen der Inkubationstemperatur erstellt wird. Die Normalisierung kann beispielsweise mittels Subtraktion oder Division durchgeführt werden.

Durch eine frühzeitige Referenzmessung lassen sich Entwicklungsunterschiede im Embryo besser erfassen. Auch dies ist besonders für eine möglichst frühe Erkennung des Geschlechts eines gegebenen Embryos von großem Vorteil.

In einem weiteren Aspekt der vorliegenden Erfindung umfasst das Verfahren eine Eichmessung zur Kalibrierung der Sensoreinheit, wobei die Eichmessung unter Abdeckung des Sensors und/oder an einem Referenzobjekt, beispielsweise einem Teflon-Referenzblock, durchgeführt wird, und wobei die Eichmessung vorzugsweise automatisch durchgeführt wird.

Die Genauigkeit der Messungen kann durch eine Eichung des Sensors stark erhöht werden. Eine Abdeckung des Sensors ermöglicht dabei eine effektive Messung des sogenannten "dark noise"-Verhaltens des Detektors, also Messung von Ereignissen, ohne ursächlichen externen Input. Die Verwendung von Teflon-Referenzblöcken bietet insbesondere den Vorteil, dass Teflon lediglich zu einer bekannten Dämpfung der Amplitude eingehender Strahlung bewirkt, und dabei die spektrale Verteilung im Wesentlichen invariant lässt, was ebenfalls zu Kalibrierung der Sensorik dienlich sein kann.

Gemäß einem weiterführenden Aspekt der Erfindung wird zur Bestimmung des Geschlechts des Embryos ein Spektralbereich der Referenz- und Messspektren in einem Wellenlängenbereich zwischen 520 nm und 580 nm, vorzugsweise zwischen 540 nm und 575 nm, weiter vorzugsweise zwischen 520 nm und 680 nm, weiter vorzugsweise zwischen 520 nm und 870 nm genutzt. Dieser Spektralbereich kann auch als Nutzsignal bei der oben beschriebenen Bewertung aufeinanderfolgender Messungen bei Bestrahlung aus unterschiedlichen Richtungen genutzt werden.

Die Wahl des relevanten Frequenzbereichs erleichtert die Geschlechtserkennung insbesondere durch eine genauere Auflösung diskriminierender Absorptionsbereiche. Insbesondere ist das Hämoglobinabsorptionsspektrum bei der Geschlechtserkennung des Embryos von besonderer Relevanz. Grundsätzlich ist allerdings der Bestrahlungsbereich nicht auf das sichtbare Spektrum eingeschränkt, sondern kann insbesondere auch den Infrarot- und Ultraviolett-Bereich einschließen.

Gemäß einer weiteren möglichen Fortbildung weist das Verfahren das Beziehen von Zusatzdaten auf, insbesondere aus einer dezentralen Daten-Cloud, wobei die Auswertung der Messspektren unter Berücksichtigung der Zusatzdaten erfolgt.

Die Berücksichtigung solcher Zusatzdaten ermöglicht insbesondere auch die Miteinbeziehung von Daten, welche nicht aus der eigentlichen Messung stammen, in den Klassifizierungs- und Selektionsprozess.

Gemäß einem Gedanken der Erfindung wird der Bestimmung des Geschlechts des Eis eine Konfidenz zugeordnet.

Es sei an dieser Stelle nochmals darauf hingewiesen, dass die im Rahmen des erfindungsgemäßen Messsystems beschriebenen Merkmale und Vorteile auch auf das erfindungsgemäße Verfahren zutreffen und übertragbar sind. Ebenso sind die beschriebenen Merkmale und Vorteile des Messsystems, insbesondere die Details zu dessen Zusammensetzung, auf das Verfahren anwendbar. Funktionale Merkmale, die im Rahmen des erfindungsgemäßen Messsystems beschrieben wurden, sind als Verfahrensschritte in dem erfindungsgemäßen Verfahren anwendbar. Ebenso können im Rahmen des erfindungsgemäßen Verfahrens beschriebene Verfahrensschritte in dem Messsystem angewendet werden, indem entsprechende Komponenten des Messsystems dazu ausgebildet werden, die erfindungsgemäßen Verfahrensschritte durchzuführen.

Eine bevorzugte Weiterbildung des erfindungsgemäßen Verfahrens ist ein Verfahren, wobei die Konfidenz der Geschlechtsbestimmung berücksichtigt wird, um anhand einer Vielzahl von einstellbaren externen Parametern, wie die Brutzyklus-spezifische Sterberate der Embryos, die gewünschte Outputmenge oder die gewünschte Geschlechterverteilung über eine Aussortierung von Eiern zu entscheiden.

In einem weiteren bevorzugten Aspekt der Erfindung wird anhand der Konfidenz eine Beobachtungsdauer festgelegt, und/oder werden weitere Messungen durchgeführt, um die Konfidenz zu erhöhen.

Somit können Maßnahmen zur Erhöhung der Konfidenz getroffen werden. Dies ist besonders in der frühembryonalen Phase von Vorteil, da die Geschlechtsbestimmung hier noch besonders herausfordernd und oft fehlerbehaftet ist.

Gemäß einer weiteren möglichen Fortbildung umfasst das Verfahren die gleichzeitige Messung einer Vielzahl von Eiern, insbesondere die gleichzeitige Erzeugung einer Vielzahl von Referenz- und/oder Messspektren an einer Vielzahl von Eiern, vorzugsweise mittels einer Vielzahl von Messsystemen.

Um eine möglichst effiziente Abfertigung einer großen Zahl an Eiern zu gewährleisten und eine Störung der Eier durch wiederholte oder lang andauernde Messungen zu vermeiden, ist es vorteilhaft, Messungen möglichst simultan ablaufen zu lassen. Dies wird ermöglicht, indem eine Vielzahl von Messungen gleichzeitig vorgenommen werden. Insbesondere kann dies auch eine Vielzahl von Messungen an einem Ei, zum Beispiel mit verschiedenen Sensoren oder verschiedenen Strahlungstypen, einschließen.

Eine weitere (bevorzugte) Fortbildung des erfindungsgemäßen Verfahrens liegt in der gleichzeitigen Messung einer Vielzahl von Eiern, derart durchgeführt, dass die Interferenz zwischen verschiedenen Messsystemen während der Erzeugung der Vielzahl von Referenz- und/oder Messspektren minimiert wird.

Dies vermeidet unnötige Störeinflüsse auf eine Messung aufgrund parallel ablaufender Messungen und erhöht somit die Genauigkeit der einzelnen Messungen. Gleichzeitig reduziert dies die Anforderungen an die Abschirmung der Messeinheit von Störeinflüssen, was sowohl in einer einfacheren als auch einer kostensparenden Bauweise des Gesamtsystems resultiert.

Die Erfindung wird im Folgenden auch hinsichtlich weiterer Merkmale und Vorteile anhand von Ausführungsbeispielen beschrieben, die unter Bezugnahme auf die Figuren näher erläutert werden. Hierbei zeigen:
- Fig. 1: eine Messanordnung eines Messsystems gemäß einem Ausführungsbeispiel der vorliegenden Erfindung mit getrennter Bestrahlungs- und Sensoreinheit;
- Fig. 2a: eine Messanordnung eines Messsystems gemäß einem Ausführungsbeispiel der vorliegenden Erfindung mit kombinierter Bestrahlungs- und Sensoreinheit;
- Fig. 2b: eine Variation der Messanordnung aus Fig. 2a mit einer Bestrahlungseinheit mit einer Vielzahl von Strahlungsquellen;
- Fig. 2c: eine Ansicht der Bestrahlungseinheit aus Fig. 2b von unten;
- Fig. 3: ein Messsystem gemäß einem Ausführungsbeispiel der vorliegenden Erfindung mit mehreren Bestrahlungs- und Sensoreinheiten;
- Fig. 4: ein Messsystem gemäß einem Ausführungsbeispiel der vorliegenden Erfindung mit einem Trolley und mehreren Messarmen;
- Fig. 5: eine Trolley-Transportvorrichtung sowie eine stationäre Messeinheit;
- Fig. 6: ein Messsystem gemäß einem Ausführungsbeispiel der vorliegenden Erfindung aus der Vogelperspektive;
- Fig. 7: eine Messsäule eines Messsystems gemäß einem Ausführungsbeispiel der vorliegenden Erfindung;
- Fig. 8: ein weiteres Ausführungsbeispiel der Messanordnung eines erfindungsgemäßen Messsystems;
- Fig. 9: eine Detailansicht einer Trolley-Kippvorrichtung eines Messsystems gemäß einem Ausführungsbeispiel der vorliegenden Erfindung;
- Fig. 10: eine Ansicht eines weiteren Ausfühhrungsbeispiels der Bestrahlungs- und Sensoreinheit;
- Fig. 11: ein Ausführungsbeispiel einer Trolley-Transportvorrichtung und einer Messeinheit.

Die Figuren sind lediglich schematischer Natur und dienen ausschließlich dem Verständnis der Erfindung. Gleichartige Elemente sind in der Beschreibung der Ausführungsbeispiele mit denselben Bezugszeichen versehen.

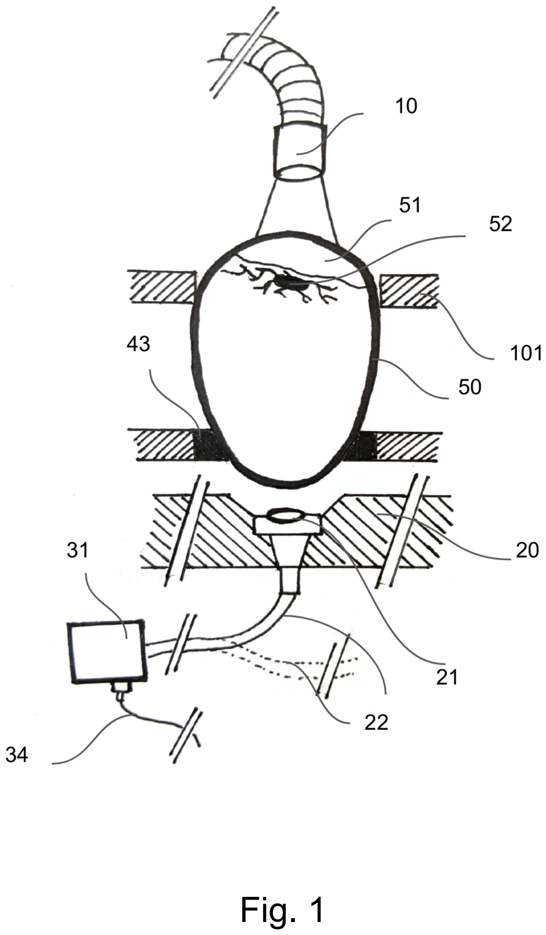

Figur 1 zeigt eine schematische Ansicht einer Messanordnung, wie sie in einem Messsystem gemäß einem Ausführungsbeispiel der vorliegenden Erfindung zum Einsatz kommt. Das dargestellte Ausführungsbeispiel eignet sich für Fälle, in denen eine Messung in Durchleuchtung durchgeführt wird. Die zum erfindungsgemäßen Messsystem gehörige Messanordnung weist eine Bestrahlungseinheit 10 auf, sowie eine Sensoreinheit 20, zwischen denen ein Ei 50 zur Messung angeordnet ist. Dabei ist die Sensoreinheit 20 auf der von der Bestrahlungseinheit 10 aus gesehen gegenüberliegenden Seite des Eis 50 angeordnet. Die Sensoranordnung 20 ist mit einem Spektrometer 31 verbunden. An dem Spektrometer 31 sind Strom- und Datenanschlüsse zur Verbindung mit einem Strom-und/oder Datenkabel vorgesehen, das in Fig. 1 schematisch mit dem Bezugszeichen 34 gezeigt ist.

Die Bestrahlungseinheit 10 ist dazu ausgebildet, Strahlung in Richtung des Eis 50 abzugeben, das in der Messanordnung angeordnet ist. Bei der Strahlung handelt es sich um elektromagnetische Strahlung wie beispielsweise (sichtbares) Licht, Infrarotstrahlung, Röntgenstrahlung oder dergleichen. Wie hier gezeigt, befindet sich ein Embryo, welcher im Wesentlichen durch die darin enthaltenen Blutgefäße 52 identifizierbar ist, unterhalb eines Lufteinschlusses 51 am von der Gravitationsrichtung aus gesehen oberen Ende des Eis 50. Die Bestrahlungseinheit 10 ist dabei üblicherweise gebildet durch eine Lichtquelle im sichtbaren Bereich, wie beispielsweise eine Glühbirne, eine LED oder eine Xenon-Lichtbogen Lampe. Bestrahlungseinheiten im Sinne der vorliegenden Erfindung sind allerdings alle Emitter elektromagnetischer Strahlung, welche geeignet sind, Absorptionsspektren des Eis 50 zu erzeugen. In dem in Fig. 1 gezeigten Ausführungsbeispiel weist die Bestrahlungseinheit 10 einen Lichtleiter auf, der zur Führung von elektromagnetischer Strahlung, die von einer (nicht gezeigten) Strahlungsquelle emittiert wird, an das Ei 50 dient.

Das Ei 50 ist mit seinem stumpfen Ende der Bestrahlungseinheit 10 zugewandt und mit seinem spitzen Ende der Sensoreinheit 20 zugewandt. Mit seinem spitzen Ende ist das Ei 50 in einem optischen Entkopplungselement 43 angeordnet, das verhindert, dass Strahlung direkt zur Sensoreinheit 20 gelangt, ohne zuvor das Ei 50 zumindest teilweise durchlaufen zu haben. Das optische Entkopplungselement 43 kann dabei die Form einer Blende oder einer Bürste annehmen, welche dazu ausgebildet ist, eng an der Eierschale anzuliegen, um eine optimale Abdichtung zu erlauben. Vorzugsweise kann dies zum Beispiel durch elastische oder anschmiegsame Materialien zur Herstellung des Entkopplungselements ermöglicht werden, oder durch eine bürstenartige Anordnung, welche am Ei 50 anliegt. Weiter vorzugsweise ist das Entkopplungselement 43 dabei in seiner Grundform an die Aufnahme des Eis 50 angepasst.

Dabei ist auch denkbar, wenngleich nicht notwendig, dass das Entkopplungselement 43 ebenfalls als Befestigungsmittel fungiert, und das Ei 50 in einer bestimmten Orientierung fixiert.

An seiner der Bestrahlungseinheit 10 zugewandten Seite ist das Ei 50 von einem Eier-Fixierungselement 101 umgeben, das ebenfalls zur Fixierung des Eis 50 in einer vorgegebenen Orientierung dient. Die Orientierung des Eis 50 kann anhand einer Achse definiert werden, die durch das spitze und das stumpfe Ende des Eis 50 verläuft und zu der die Schale des Eis 50 im Wesentlichen rotationssymmetrisch ist.

As dem Ei 50 austretende Strahlung wird durch die Sensoreinheit 20 detektiert. Zur Übertragung der an der Sensoreinheit 20 detektierten Strahlung an das Spektrometer 31 ist ein Lichtwellenleiter 22 vorgesehen. Es ist dabei vorteilhaft, obschon nicht notwendig, zur Verstärkung des empfangenen Signals dem zum Spektrometer 31 führenden Lichtwellenleiter 22 ein optisches Sammelelement 21 vorzuschalten. Beispiele für solche optischen Sammelelemente 21 sind Kollimationslinsen, Spiegel oder andere optische Lenkelemente wie zum Beispiel Fresnel-Linsen.

Nachdem die Strahlung aufgenommen wurde, wird mittels des Spektrometers 31 ein Messspektrum der erfassten Strahlung erzeugt und über das Datenkabel 34 an eine Datenverarbeitungseinheit 33 (nicht gezeigt) weitergeleitet. Die (nicht gezeigte) Datenverarbeitungseinheit 33 steht in kommunikativer Verbindung mit einer (ebenfalls nicht gezeigten) Identifikationseinheit 23.

Dabei werden ebenfalls dem Ei 50 eindeutig zuordenbare Identifikationsdaten von der Identifikationseinheit 23 erzeugt und zusammen mit dem Messspektrum an die Datenverarbeitungseinheit 33 weitergeleitet.

Bei der Messanordnung des Messsystems sind die Bestrahlungseinheit 10 und die Sensoreinheit 20 derart angeordnet, dass sie relativ zu mindestens einem Bezugspunkt des Eis 50 bei jeder Messung gleich ausgerichtet sind. Ein solcher Bezugspunkt ist zum Beispiel gegeben durch den Schwerpunkt der Schale des Eis 50, welcher, anders als der des Gesamteis, nicht durch Verschiebungen des Embryos beeinflusst wird. Vorzugsweise sind die Bestrahlungseinheit 10 und die Sensoreinheit 20 bei jeder Messung relativ zu einer Referenzachse des Eis 50 gleich ausgerichtet. Als Referenzachse des Eis 50 kann die vorstehend beschriebene Symmetrieachse, die durch die Pole des Eis verläuft, genutzt werden. Eine Änderung der Lage der Referenzachse des Eis 50 äußert sich in einer Verkippung des Eis 50.

Es ist möglich, bei einer gleichbleibenden Ausrichtung der Bestrahlungseinheit 10 und der Sensoreinheit 20 relativ zu den genannten Bezugspunkten oder der Referenzachse des Eis 50 die gesamte Anordnung aus dem Ei 50 und der Messanordnung zu verkippen. Das ermöglicht Messungen bei verschiedenen Kippstellungen des Eis 50 bei gleichbleibender relativer Anordnung der Bestrahlungseinheit 10 und der Sensoreinheit 20 relativ zum Ei 50. Dadurch kann der Embryo durch eine kontrollierte Kippung aus dem Beleuchtungsfeld der Bestrahlungseinheit 10 bzw. dem Sichtfeld der Sensoreinheit 20 entfernt werden, um beispielsweise eine Referenz- oder Kalibrierungsmessung durchzuführen. Es wird also stets das Ei 50 mitsamt der Bestrahlungseinheit 10 und der Sensoreinheit 20 zusammen verkippt.

Alternativ kann auch eine Vielzahl an Messungen jeweils bei verschiedenen Kippstellungen des Eis 50 vorgenommen werden. Um die aktuelle Kippstellung des Eis 50 zu ermitteln, wird dabei ein Winkel zwischen einer vordefinierten Referenzachse des Eis 50 und einer Referenzachse des Messsystems bestimmt. Die Referenzachse des Messsystems wird im Rahmen der vorliegenden Beschreibung auch als Bezugsachse bezeichnet. Ein Beispiel für eine solche Bezugsachse wäre beispielsweise die Verbindungslinie zwischen der Bestrahlungseinheit 10 und der Sensoreinheit 20 in Figur 1. Ein weiteres Beispiel wäre eine Bezugsachse in Richtung des Gravitationsfeldes.

Es ist dabei nicht wesentlich, auf welche Art der Winkel zwischen der Referenzachse und der Bezugsachse bestimmt wird. Denkbar ist eine mechanische Erfassung, durch das Voreinstellen eines Winkels des Eier-Trays, in welchem die Eier fixiert sind, oder eine optische Ermittlung des Winkels, zum Beispiel durch Erkennung der Ei-Umrisse oder durch ein am Eier-Tray montiertes Gyroskop.

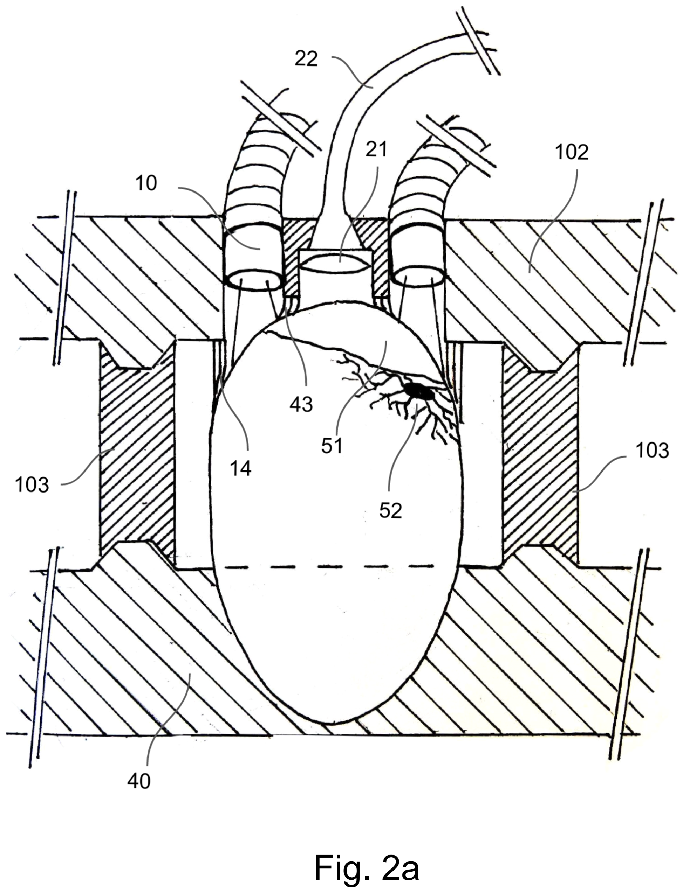

Figur 2a zeigt ein weiteres Ausführungsbeispiel der Messanordnung eines erfindungsgemäßen Messsystems, bei der die Bestrahlungseinheit 10 und die Sensoreinheit 20 auf derselben Seite des Eis 50 angeordnet sind. In diesem Ausführungsbeispiel wird durch die Sensoreinheit nicht durch das Ei 50 hindurchgetretene Strahlung aufgenommen, sondern im Ei 50 zurück gestreute Strahlung. Der Aufbau mit einer Anordnung der Bestrahlungseinheit 10 und der Sensoreinheit 20 auf der gleichen Seite des Eis 50 ermöglicht dabei eine einfachere Kippung des Eier-Trays 40, da die Relativposition der Bestrahlungs- und Sensoreinheit 10, 20 zum Ei 50 automatisch konstant bleibt. Eine solche Anordnung ist insbesondere von Vorteil, wenn das Messsystem einen Messaufsatz 102 aufweist, welcher auf ein bereits bestehendes Eier-Tray 40 aufgesetzt werden kann. Um den Anbringungsaufwand zu minimieren, und gegebenenfalls auch für eine optische Entkopplung von benachbarten Messsystemen zu sorgen, kann ein derartiger Messaufsatz 102 speziell an das Eier-Tray 40 angepasste Messaufsatz-Adapter 103 aufweisen.

Die Messaufsatz-Adapter 103 sind derart ausgestaltet, dass sie in ein Eier-Tray eingreifen oder es in Teilen in sich aufnehmen, um eine lösbare Verbindung zwischen dem Messaufsatz 102 und dem Eier-Tray 40 zu ermöglichen, oder darauf aufgesetzt zu werden.

Der Messaufsatz-Adapter dient zusätzlich als Adapter zwischen einer Vielzahl an unterschiedlichen, in der Praxis üblichen, Eier-Trays und dem Messaufsatz 102. Dadurch minimiert sich die benötigte Varianz des komplexeren Messaufsatzes 102.

Durch das vorsehen von Belüftungsöffnungen 106 (hier nicht gezeigt) wird verhindert, dass das Ei 50 nicht ausreichend in Wärmeaustausch mit der Inkubatorluft steht.

Ein derartiger Messaufsatz kann dabei automatisch oder manuell zwischen verschiedenen Eier-Trays 40 bewegt werden, um Messungen vorzunehmen.

Um einen maximalen Anteil der von der Bestrahlungseinheit 10 emittierten Strahlung in das Ei einzuleiten, ist es von Vorteil, Strahlbegrenzer 14 zu verwenden, welche eine zu große Ausbreitung des von der Bestrahlungseinheit 20 ausgesandten Lichtkegels verhindern. Die Strahlbegrenzer können dabei ebenfalls durch Blenden oder Bürsten gegeben sein. Zudem verhindern sie, dass angrenzende Messsysteme durch Streulicht gestört werden.

Um den maximalen Informationsgehalt auch bei einer nicht mittigen Ausrichtung des Embryos 52 zu erlangen, sieht man in dem Ausführungsbeispiel gemäß Fig. 2a, dass die Bestrahlungseinheit 10 mehrere Bestrahlungseinheiten 10a aufweist. Dies ist von Vorteil, um den Embryo bestmöglich ins Sichtfeld zu bekommen um einen großen Einfluss des Embryos auf das Spektrum sicherzustellen.

Da die Blutgefäße 52 bei der Entwicklung des Eis 50 häufig nicht mittig anwachsen, sondern an einer Seite des stumpfen Endes des Eis 50, wie in Fig. 2a gezeigt, ist es ferner vorteilhaft, eine Bestrahlungseinheit 10 mit einer Vielzahl von Strahlungsquellen 10a zu verwenden, bei der die einzelnen Strahlungsquellen 10a individuell ansteuerbar bzw. aktivierbar sind. In dem in Fig. 2a gezeigten Beispiel ist bei einer Aktivierung der linken Strahlungsquelle 10a ein sehr geringes Nutzsignal zu erwarten, während die Aktivierung der rechten Strahlungsquelle 10a, die genau oberhalb der Blutgefäße 52 angeordnet ist, ein starkes Nutzsignal zu erwarten ist.

Bei Verwendung einer Bestrahlungseinheit 10 mit einer Vielzahl von individuell aktivierbaren Strahlungsquellen 10a kann die Erzeugung eines (Referenz- oder Mess-) Spektrums derart erfolgen, dass zunächst Messungen durchgeführt werden, bei denen jeweils nur eine Strahlungsquelle 10a (oder nur ein Teil der Strahlungsquellen 10a) aktiviert ist. Aus den erhaltenen Messungen können dann eine oder mehrere Messungen mit vergleichsweise hohem Nutzsignal selektiert werden. Werden mehrere Messungen mit vergleichsweise hohem Nutzsignal selektiert, können diese miteinander in geeigneter Weise verrechnet, beispielsweise gemittelt, werden, um ein optimiertes Spektrum zu erhalten. Ebenso ist es möglich, die Messung(en) mit vergleichsweise hohem Nutzsignal mit Messungen mit vergleichsweise niedrigem Nutzsignal zu verrechnen, um das Signalzu-Rausch-Verhalten zu verbessern. Hierbei ist es beispielsweise denkbar, die Messung mit hohem Nutzsignal durch eine Messung mit schwachem Nutzsignal zu teilen, oder die Messung mit schwachem Nutzsignal von der Messung mit hohem Schutzsignal zu subtrahieren. Als Messung mit schwachem Nutzsignal kann die Messung mit dem vergleichsweise schwächsten Nutzsignal selektiert werden, oder es können mehrere Messungen mit vergleichsweise schwachem Nutzsignal gemittelt werden.

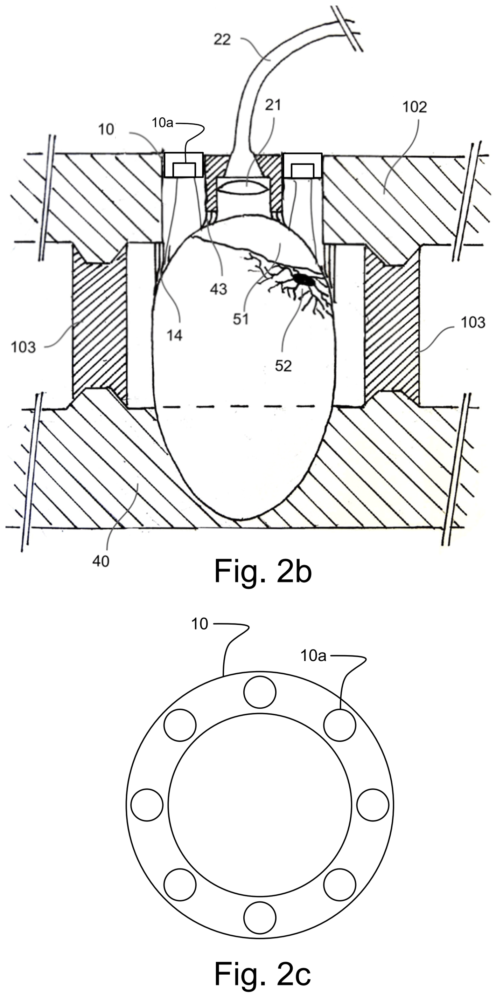

Fig. 2b zeigt eine Modifikation des Ausführungsbeispiels aus Fig. 2a, bei der als Bestrahlungseinheit 10 eine Ringleuchte verwendet wird, die eine Vielzahl von Strahlungsquellen 10a aufweist, die auf einem Ring angeordnet sind. Dies stellt eine konstruktiv einfache Lösung zur Bestrahlung des Eis 50 aus einer Vielzahl von Richtungen dar. Der Durchmesser der Ringleuchte ist kleiner gewählt als der Durchmesser des Eis 50 an seiner breitesten Stelle senkrecht zur Symmetrieachse, um sicherzustellen, dass jede Strahlungsquelle 10a das Ei 50 durchleuchtet. Besonders bevorzugt ist die Verwendung einer Ring-LED. Fig. 2c zeigt schematisch den Aufbau der Ringleuchte aus Fig. 2b. Die Ringleuchte weist 8 Strahlungsquellen 10a auf, die in einer ringförmigen Anordnung an der Ringleuchte vorgesehen sind.

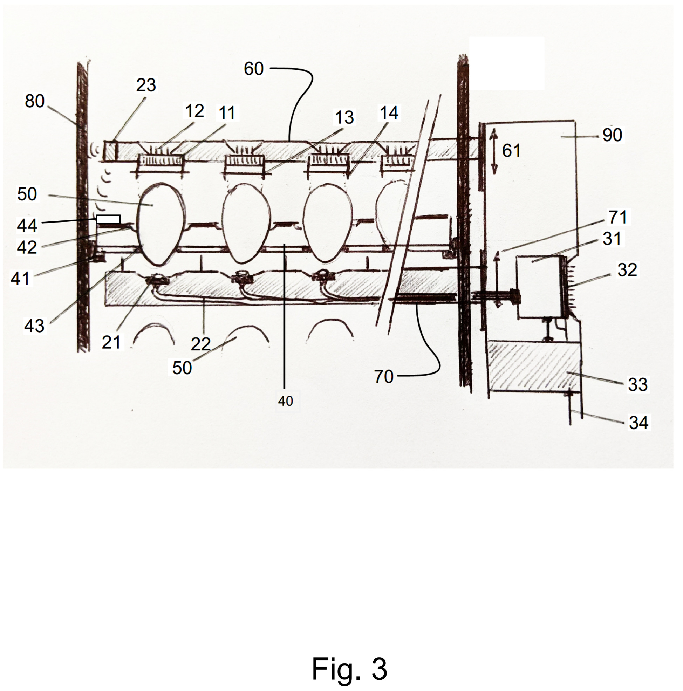

Figur 3 zeigt ein weiteres Ausführungsbeispiel der vorliegenden Erfindung, bei dem das Messsystem mehrere Messanordnungen aufweist. Als Messanordnung sind dabei die jeweiligen Paare aus Bestrahlungseinheit mit Lichtquelle 11 und Sensoreinheit mit optischem Sammelelement 21 zu verstehen. Die mehreren Messanordnungen können simultan betrieben werden, um den Messdurchsatz zu erhöhen. Die Sammelelemente 21 sind jeweils mit Lichtwellenleitern 22 versehen, die mit einem Spektrometer 31 zur Detektion der durch die Eier 50 transmittierten Strahlung und der Erzeugung entsprechender Spektren verbunden sind. Das Spektrometer 31 ist mit einer Datenverarbeitungseinheit 33 verbunden, die vom Spektrometer 31 erzeugte Spektren gemeinsam mit Identifikationsdaten speichert, die eine Zuordnung der Spektren zu den einzelnen Eiern 50 im vermessenen Eier-Tray 40 erlaubt. Die Datenverarbeitungseinheit weist Anschlüsse für Strom- und Datenkabel auf, die in Fig. 3 schematisch dargestellt und mit dem Bezugszeichen 34 bezeichnet sind. Über die Anschlüsse 34 kann die Datenverarbeitungseinheit 33 mit einer (in Fig. 3 nicht dargestellten) Klassifikationseinheit 35 verbunden werden, die dazu ausgebildet ist, das Geschlecht des Embryos in den einzelnen Eiern 50 auf Grundlage mindestens eines Referenzspektrums und mindestens eines Messspektrums zu bestimmen. Die Klassifikationseinheit 35 kann auch in der Datenverarbeitungseinheit 33 integriert sein.

Die Lichtquellen 11 sind in einem ersten Messarm 60 eingebracht, und die zu den Sensoreinheiten gehörigen Sammelelemente 21 in einen zweiten Messarm 70. Gemäß diesem Ausführungsbeispiel werden der erste Messarm 60 über und der zweite Messarm 70 unter einem Eier-Tray 40 positioniert, welches innerhalb eines Inkubators 80 auf einer Eier-Tray-Auflage 41 angebracht ist. Dabei befinden sich die Eier 50 in Eierausnehmungen 42 des Eier-Trays 40. Es ist aber auch ebenso denkbar, die Messarme neben dem Ei oder in einer sonstigen Stellung anzubringen, solange zueinander gehörige Bestrahlungseinheiten 10 und Sensoreinheiten 20 jeweils ein Ei zwischen sich aufnehmen und die Relativposition der Messanordnung zum Ei konstant bleibt, bzw. bei Kippungen des Eis mitgedreht wird.

Da es bei der Verwendung einer Vielzahl von Lichtquellen, wie beispielsweise LEDs, zu einer verstärkten Wärmeentwicklung kommen kann, welche sich sowohl nachteilig auf die Temperierung des Inkubators als auch auf die Lebensdauer der Lichtquellen auswirken kann, sind die Lichtquellen 11 mit Kühlelementen 12 versehen. Die Kühlelemente 12 können dabei aktiv oder passiv zur Kühlung beitragen. Beispielsweise sind Kühlrippen dazu geeignet, einen schnelleren Temperaturaustausch zu fördern. Gleiches gilt für das Kühlelement 32 des zur Messung der Spektren verwandten Spektrometers 31.

Zusätzlich können die Messarme 60, 70 Belüftungsöffnungen aufweisen, um eine möglichst gute Ventilation um das Ei 50 herum zu gewährleisten.

Zusätzlich zu den vorstehend genannten Strahlbegrenzern kann es weiterhin vorteilhaft sein, bereits die Bestrahlungseinheit mit einem optischen Lenkelement 13 wie beispielsweise einer asphärischen Linse, einer Fresnel-Linse oder ähnlichem auszustatten, um möglichst viel der von den Lichtquellen 11 emittierten Strahlung in das Ei 50 einzukoppeln. Ferner sind in dem in Fig. 3 gezeigten Ausführungsbeispiel Strahlbegrenzer 14 an den einzelnen Beleuchtungseinheiten vorgesehen, um zu verhindern, dass die von den einzelnen Lichtquellen 11 emittierte Strahlung in benachbarte Messanordnungen streut.

Um eine gegenseitige Beeinflussung der Messsysteme so gering wie möglich zu halten, können zusätzliche Maßnahmen getroffen werden. Beispielsweise kann das Messsystem derart konfiguriert sein, dass eine Messung in mehreren, beispielsweise zwei Schritten erfolgt, wobei in einem ersten Schritt nur jede zweite Messanordnung eine Messung vornimmt und anschließend in einem zweiten Schritt die übrigen Messanordnungen ihre Messung vollziehen, so dass immer mindestens ein Ei 50 Abstand zwischen aktiven Messanordnungen liegt.

Um die Messsysteme einfach zwischen den mehreren in einem Trolley befindlichen Eier-Trays hin und her zu bewegen, weist der erste Messarm 60 einen ersten Bewegungsmechanismus 61 und der zweite Messarm 70 einen zweiten Bewegungsmechanismus 71 auf. Diese dienen dazu, die Position der Messarme 60, 70 vertikal und/oder horizontal, relativ zu einer Messsäule 90, welche die Messarme trägt, zu verschieben. Somit können nacheinander alle Eier-Trays innerhalb eines Trolleys einer Messung unterzogen werden. Vorzugsweise ist für den Transport der Eier-Trays zum Messsystem ein Mechanismus zum Entnehmen von Eier-Trays aus den Eier-Trolleys im Inkubator vorgesehen, der die Eier-Trays nach der Messung auch wieder in den Eier-Trolley einsetzen kann. Dadurch kann ein größerer Bauraum für die Messeinheit zur Verfügung gestellt werden.

Um die gegenwärtige Position der Messanordnungen zu bestimmen und eine eindeutige Zuordnung der Referenz- und Messspektren zu den verschiedenen Eiern sicherzustellen, weist das Messsystem eine Identifikationseinheit 23 auf. Die Identifikationseinheit 23 ist dazu ausgelegt, ein Eier-Tray oder einen Trolley beispielsweise über angebrachte Marker oder Sensoren zu identifizieren. Um eine eindeutige Zuordnung der Messspektren zu den Eiern 50 zu gewährleisten, sendet die Identifikationseinheit 23 Daten, wie zum Beispiel einen Identifier eines Trolleys, eines Eier-Trays 40 und einer bestimmten Ei-position innerhalb des Eier-Trays 40 an die Datenverarbeitungseinheit 33, welche die Identifikationsdaten eines Eis 50 zusammen mit den gemessenen Referenz- und/oder Messspektren ablegt. Die einzelnen Eier-Trays 40 sind anhand eines Eier-Tray-Identifikationsmerkmals 44 identifizierbar, das durch optisch auslesbare Codes, beispielsweise Barcodes oder QR-Codes, oder durch RFID-Tags gebildet sein kann. Die Identifikationseinheit 23 ist entsprechend geeignet als optisches Lesegerät oder als RFID-Reader ausgebildet.

In einer anderen Ausgestaltung kann eine Identifikation von Trolleys, Eier-Trays 40 oder Eiern 50 durch eine Weitergabe von Daten an die Datenverarbeitungseinheit 33 erfolgen, die Informationen über die aktuelle Position der Messarme 60, 70 bzw. der Bewegungsmechanismen 61, 71 enthalten. Aus der Position der Messarme 60, 70 kann auf die Position der Messanordnungen geschlossen werden und so ermittelt werden, welche Eier einer Messung unterzogen wurden.

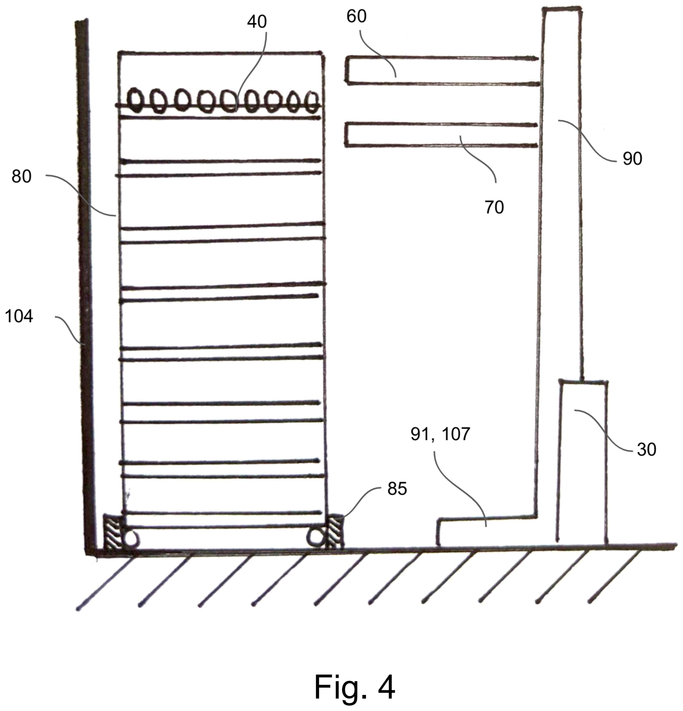

Figur 4 zeigt eine Außenansicht eines Messsystems des in Fig. 3 gezeigten Typs, mit dem es möglich ist, Eier direkt in einem Eier-Tray zu vermessen, das in einem Trolley angeordnet ist. In Fig. 4 ist ein Trolley 80 mit einer Vielzahl von Eier-Trays 40 dargestellt. Das Messsystem und der Trolley 80 befinden sich in einem Inkubator. Die Inkubatorwand ist in Fig. 4 mit dem Bezugszeichen 104 bezeichnet.

Das Messsystem weist einen ersten Messarm 60, einen zweiten Messarm 70 und eine Messsäule 90 auf. Die Messsäule 90 ist auf einem Säulenfuß 91 angeordnet. Der Säulenfuß 91 weist eine Transportvorrichtung 107 zum Transport der Messsäule 90 innerhalb des Inkubators 104 zwischen verschiedenen Trolleys 80 auf. Es ist auch denkbar, die Transportvorrichtung 107 zum Transport des Messsystems zwischen verschiedenen Inkubatoren zu verwenden. In dem in Fig. 4 gezeigten Ausführungsbeispiel ist eine Auswertungseinheit 30 in der Messsäule 90 integriert, die ein Spektrometer, eine Datenverarbeitungseinheit und/oder eine Klassifikationseinheit enthalten kann, oder Kommunikationsmittel, um eine kommunikative Verbindung zu den genannten Einheiten herzustellen.

Um eine Ortung der Trolleys innerhalb des Inkubators zu ermöglichen oder zu vereinfachen, ist am Boden eines Inkubators eine Trolley-Positionierungsvorrichtung 85 vorgesehen, welche sicherstellt, dass jeder Trolley 80 an einer vorbestimmten Stelle innerhalb eines Inkubators positioniert ist. Dies kann über spezielle Halterungsvorrichtungen oder aber durch Markierungen erfolgen, welche einem Benutzer erlauben, den Trolley 80 korrekt zu positionieren.

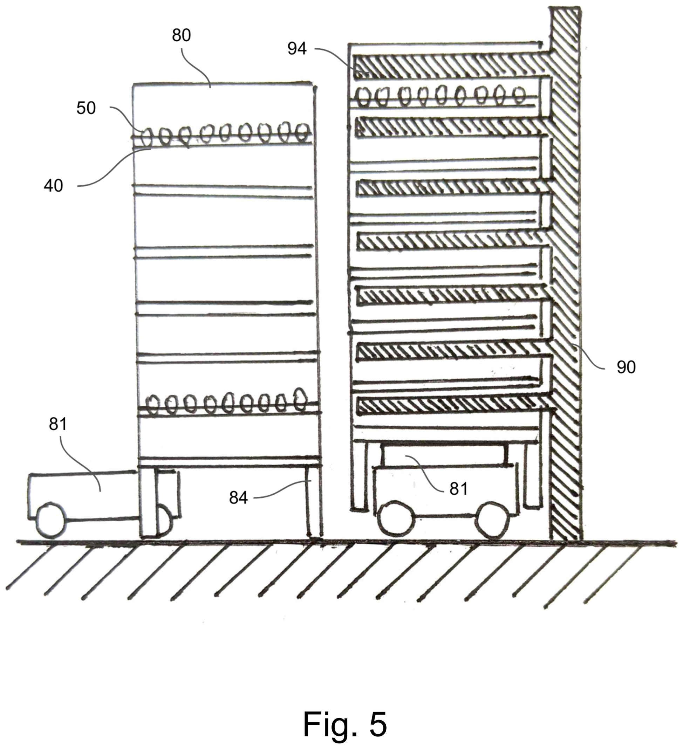

Figur 5 zeigt ein weiteres Ausführungsbeispiel der vorliegenden Erfindung, bei der eine Messsäule 90 im Inkubator fixiert ist, so dass Eier-Trolleys 80 zur Messsäule gefahren werden können. An der Messsäule 90 ist eine Vielzahl von Messarmen 94 angebracht, in denen Messanordnungen gemäß den in Fig. 1, 2a, 2b oder 3 gezeigten Ausführungsbeispielen angebracht sind. Der Transport der Eier zum Messsystem erfolgt mittels einer Trolley-Transportvorrichtung 81, welche den Eier-Trolley 80 als Ganzes zur Messsäule transportiert. Die Trolley-Transportvorrichtung 81 kann dabei vorzugsweise die Gestalt eines Roboters haben, welcher selbstständig die Trolley-Positionen abfährt, und die Trolleys 80 zu dem fixierten Messsystem fährt. Die Trolley-Transportvorrichtung 81 hat eine geringere Höhe als die Trolley-Füße 84, so dass die Trolley-Transportvorrichtung 81 unter die Trolleys 80 fahren kann. In der Trolley-Transportvorrichtung ist ein ausfahrbares Hebemittel vorgesehen, mittels dem der Eier-Trolley 80 angehoben wird und dann transportiert werden kann.

Um eine Einstellung verschiedener Kippwinkel zu erreichen, kann die Trolley-Transportvorrichtung 81 eine Trolley-Kippvorrichtung 82 aufweisen (hier nicht gezeigt), welche den Kippzustand der Eier-Trays 40 einstellt. Dazu greift sie entweder in einen bereits bestehenden Schwenkmechanismus des Eier-Trolleys 80 ein, oder sie kippt den Eier-Trolley 80 als Ganzes.

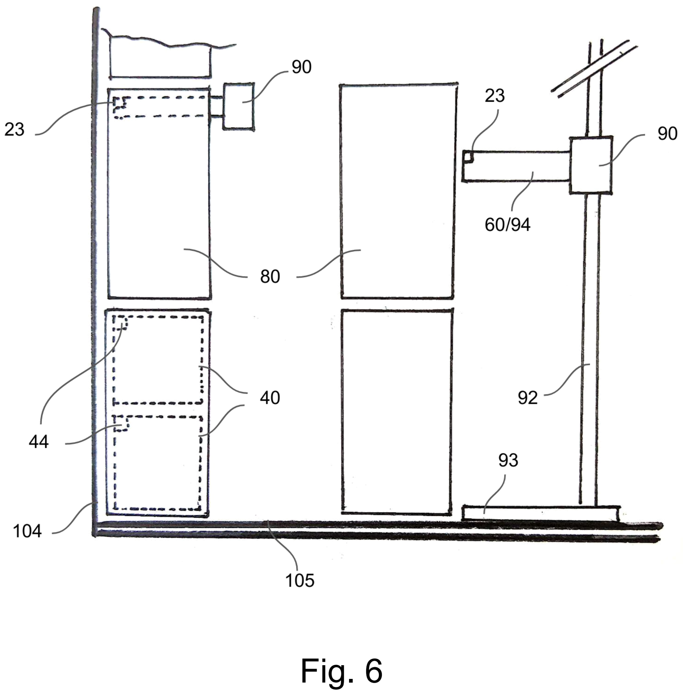

Figur 6 zeigt das Innere eines Inkubators mit mehreren Messsystemen mit einem oder mehreren Messarmen 60, 70 aus der Vogelperspektive. Der Inkubator ist durch eine Inkubatorwand 104 und eine Inkubatortür 105 begrenzt. Die Messsysteme weisen wiederum Messsäulen 90 auf. Dabei verfügt die in Fig. 6 rechts dargestellte Messsäule 90 sowohl über eine erste Horizontal-Linearführung 92 als auch über eine zweite Horizontal-Linearführung 93. Diese dienen dazu, die Position des Messarms 94 oder der Messarme 60, 70 in einer Ebene orthogonal zur Messsäule 90 einzustellen. Der Messarm 94 kann wiederum Messanordnungen gemäß den in Fig. 1, 2a, 2b oder 3 gezeigten Ausführungsbeispielen aufweisen.

Die erste Horizontal-Linearführung 92 und die zweite Horizontal-Linearführung 93 können dabei mit dem ersten Bewegungsmechanismus eines ersten Messarms 60 verbunden sein, und eine dritte Horizontal-Linearführung 92 und eine vierte Horizontal-Linearführung 93 (die in Fig. 6 nicht sichtbar sind, da sie sich genau unterhalb der ersten und zweiten Horizontal-Linearführung befinden) kann dabei mit dem zweiten Bewegungsmechanismus eines zweiten Messarms 70 verbunden sein, um eine Einstellung der Position des Messsystems in allen drei Raumrichtungen zu ermöglichen. Insbesondere können dabei der erste und zweite Messarm unabhängig voneinander positioniert werden, um beispielsweise den Winkel zwischen dem Messsystem und dem Ei einzustellen.

Auf diese Weise kann das Messsystem zwischen mehreren Trolleys 80 hin und hergefahren werden. Wie in Fig. 6 gezeigt ist, weisen die Messarme eine Identifikationseinheit 23 auf, die dazu ausgebildet ist, die Eier-Tray-Identifikationsmittel 44 auf den Eier-Trays 40 auszulesen, um eine eindeutige Zuordnung der Eier-Trays 40 oder Trolleys 80 vornehmen zu können. Aus den Koordinaten der Horizontal-Linearführung kann dann eine eindeutige Position eines Eis in einem zuvor identifizierten Eier-Tray 40 bzw. Trolley 80 ermittelt werden, um die Identität des Eis einer Messung zuzuordnen.

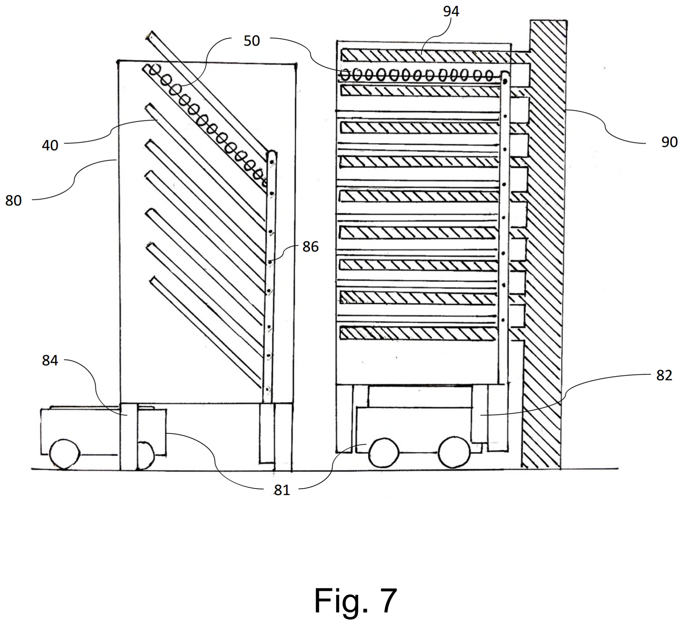

Figur 7 zeigt erneut ein Ausführungsbeispiel der vorliegenden Erfindung, bei dem eine Messsäule 90 im Inkubator fixiert ist, so dass der Trolley 80 zur Messsäule gefahren werden kann. Um eine Einstellung verschiedener Kippwinkel zu erreichen, greift hier die Trolley-Transportvorrichtung 81 in eine Trolley-Kippvorrichtung 82 ein, nachdem sie neben dem Trolley-Fuß 84 in Position gebracht wurde. In der gezeigten Ausführungsform werden die Eier-Trays 40 um eine Achse am distalen Ende der Eier-Trays um einen Kippmechanismus 86 wie ein Kipplager nach oben geschwenkt. Da die relative Ausrichtung der Messanordnungen zum Ei 50 dabei gleichbleiben soll, sind die Messarme 94 ebenfalls schwenkbar gelagert. Die Lagerposition der Messarme 94 ist auf die Position der Kippmechanismen 86 abgestimmt, so dass die Achsen der Lager der Messarme und der Kippmechanismen möglichst übereinanderliegen, wenn der Trolley 80 an der Messsäule 90 platziert ist, um ein gemeinsames Kippen der Messarme 94 und der Eier-Trays 40 zu ermöglichen. Es ist ebenso denkbar, die Eier-Trays zur Seite oder in anderer Art und Weise zu kippen, so lange dabei die Relativposition zu den Messanordnungen nicht geändert wird.

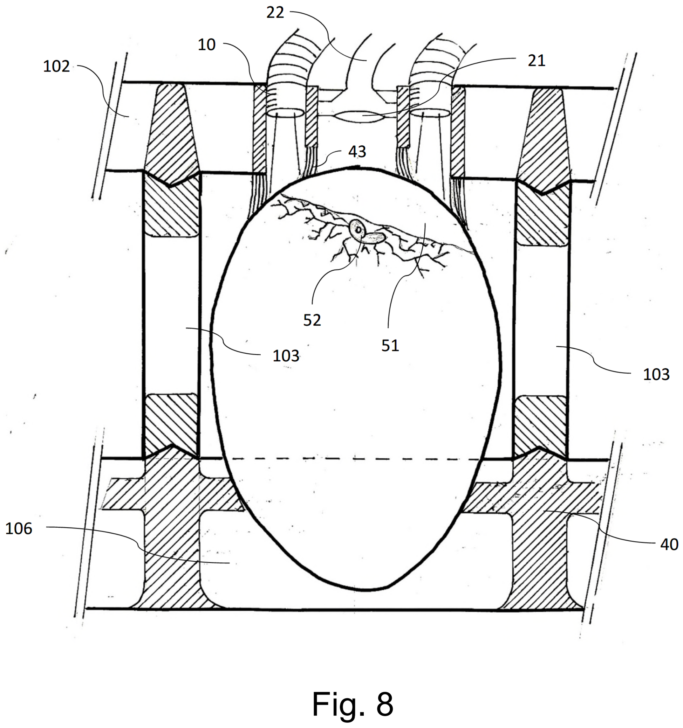

Figur 8 zeigt ein weiteres Ausführungsbeispiel einer Messanordnung eines erfindungsgemäßen Messsystems vom in Fig. 2a und 2b gezeigten Typ, bei der die Bestrahlungseinheit 10 und die Sensoreinheit 20 auf derselben Seite des Eis 50 angeordnet sind. Der gleichseitige Aufbau ermöglicht dabei eine einfachere Kippung des Eier-Trays 40, da die Relativposition der Bestrahlungs- und Sensoreinheit 10, 20 zum Ei 50 automatisch konstant bleibt. Eine solche Anordnung ist insbesondere für einen Messaufsatz 102 von Vorteil, welcher einfach auf ein bereits bestehendes Eier-Tray 40 aufgesetzt werden kann. Um den Anbringungsaufwand zu minimieren, und gegebenenfalls auch für eine optische Entkopplung von benachbarten Messsystemen zu sorgen, besitzt ein derartiger Messaufsatz 102 speziell an das Eier-Tray 40 angepasste Messaufsatz-Adapter 103. Hierbei ist es, wie gezeigt, von Vorteil, wenn die Bauteile der Messanordnung und des Eier-Trays 40 möglichst gerüstartig und/oder wabenartig ausgestaltet sind, so dass sich möglichst große Belüftungsöffnungen 106 bilden, über welche das Ei 50 in Wärmeaustausch mit der Inkubatorluft steht.

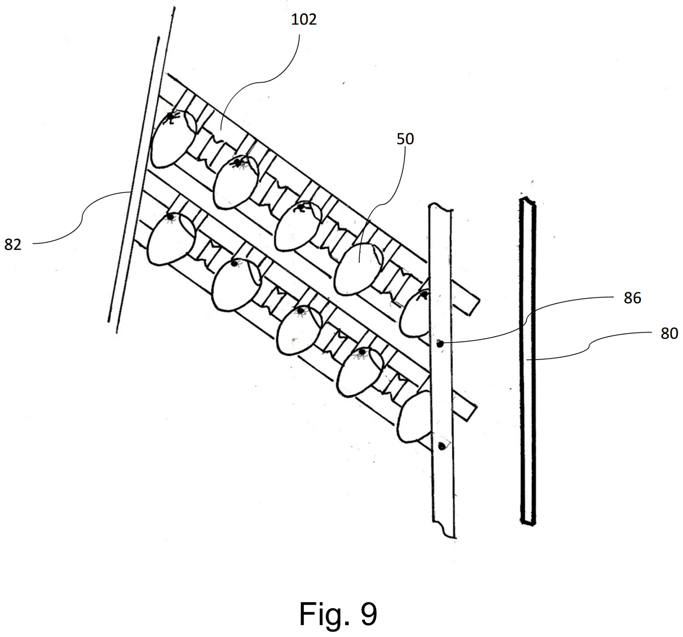

Figur 9 zeigt eine weitere Möglichkeit zur Realisierung eines Kippmechanismus 86 für die Eier-Trays. Hierbei wird durch das Anheben und Senken der Eier-Trays 40 an einem Ende mittels einer schematisch dargestellten Kippvorrichtung 8 eine Rotation um den als Kipplager ausgebildeten Kippmechanismus 86 bewirkt. Insbesondere wenn ein Messaufsatz 102 gemäß einer der in Figuren 2 und 8 gezeigten Ausführungsbeispiele verwendet wird, ist dabei darauf zu achten, dass der Abstand der Eier-Trays vertikaler Richtung ausreichend groß ist, um ein Blockieren zu verhindern, und überdies Kollisionen mit der Außenwand des Trolleys 80 zu verhindern.

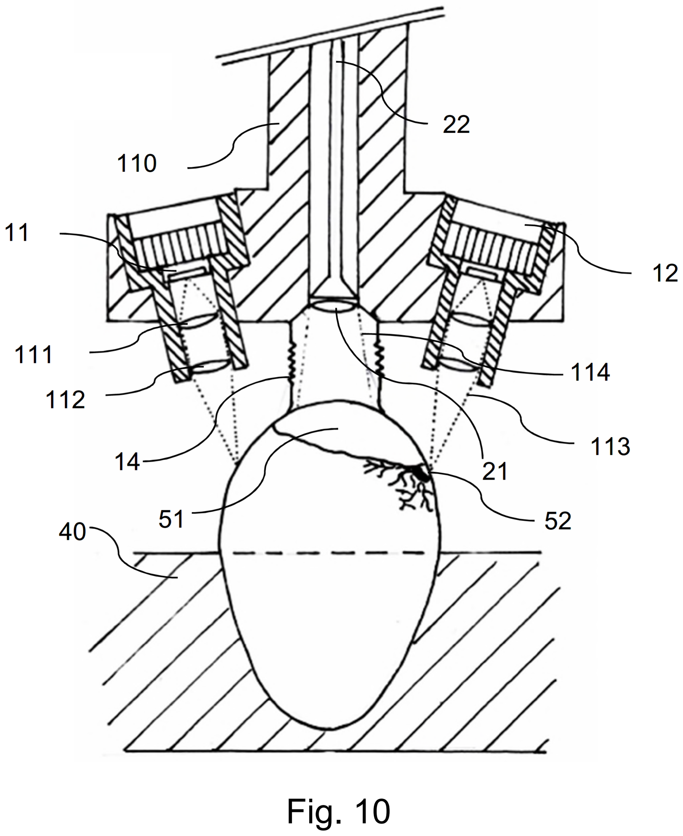

In Figur 10 ist ein weiteres Ausführungsbeispiel gezeigt. Hier ist die Anordnung der Lichtquellen 11 so ausgelegt, dass die Strahlen in einem zur Vertikalen geneigten Winkel auf das Ei 50 auftreffen, und somit zumindest teilweise seitlich eintreten. Dabei werden die aus der Lichtquelle 11 austretenden, im Wesentlichen radial in alle Richtungen abgegebenen, Strahlen durch eine erste Konvexlinse 111 und eine zweite Konvexlinse 112 so fokussiert (bzw. gebündelt), dass der resultierende Lichtkegel 113 seine Spitze auf der Eierschale hat. Die erste Konvexlinse 111 und die zweite Konvexlinse 112 sind enstprechend derart angeordnet, dass von der Lichtquelle 11 abgegebenes Licht schräg in das Ei 50 eintritt; vorzugsweise unter einem Winkel zur Vertikalen von mehr als 20°.

Das aus dem Ei 50 austretende Licht nimmt dann die Form eines diffusen Lichtstrahls 114 an, welcher durch das optische Sammelelement 21, hier eine Kolliminationslinse, wiederum gebündelt und in den Lichtwellenleiter 22 eingekoppelt wird.

Dabei werden die Lichtquellen 11 und der Lichtwellenleiter 22 durch ein Messkopfgehäuse 110 gehalten. Besonders bevorzugt ist dabei eine Anordnung, bei der die die Lichtquellen 11, wie in Figur 2c gezeigt, ringförmig angeordnet sind, wobei die entsprechenden Lichtkegel 113 dann jeweils zum Zentrum der ringförmigen Anordnung hin nach innen geneigt sind.

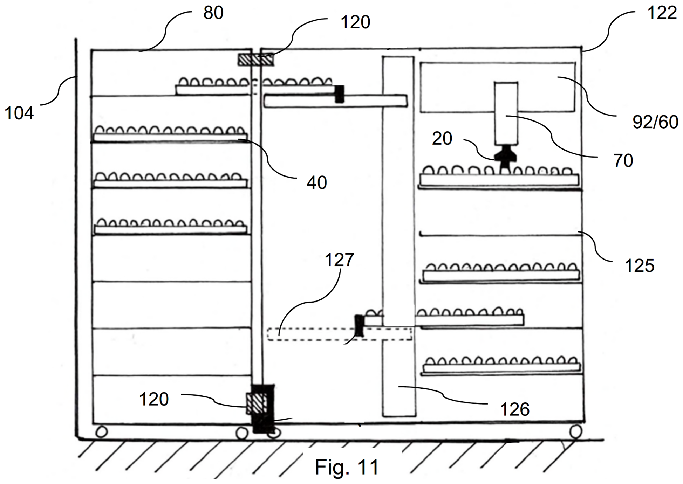

In Figur 11 ist ein weiteres Ausführungsbeispiel gezeigt, welches einen Eier-Trolley 80 in einem Inkubator zeigt. Dabei steht der Eier-Trolley 80 an der Inkubatorwand 104, gegenüber einer mobilen Eier-Messeinheit 122, welche als Ganzes zwischen verschiedenen Inkubatoren bewegt werden kann. Selbstverständlich ist es auch möglich, die mobile Eier-Messeinheit 122 hinter dem Eier-Trolley 80 im Inkubator zu platzieren. Es ist lediglich maßgeblich, dass die mobile Eier-Messeinheit 122 neben dem Eier-Trolley 80 angeordnet ist.

Die mobile Eier-Messeinheit 122 dabei so positioniert, dass ein Tray-Schlitten 127 entlang einer (im Wesentlichen horizontalen) x-Achse bewegt werden kann, um ein Eier-Tray 40 aus dem Eier-Trolley 80 zu entnehmen, und anschließend in dafür vorgesehene Puffer-Trays 125 der mobilen Eier-Messeinheit 122 abzulegen.

Innerhalb der Messeinheit 122 ist die Sensoreinheit 20 sowohl entlang der x-Achse, insbesondere entlang der/des zweiten Horizontal-Linearführung/Messarms 93/60 als auch entlang der (im Wesentlichen vertikalen) z-Achse beweglich, sodass sich die Sensoreinheit 20 zu jedem Ei des obersten Puffer-Trays 125 bewegen lässt, um eine Messung vorzunehmen. Die Bewegung entlang der x-Achse erfolgt dabei entlang einer Linearführung, bzw. entlang eines zweiten Messarms 70. Sobald das oberste Eier-Tray 40 fertig vermessen ist, lässt es sich mit einem weiteren Tray aus dem Eier-Trolley 80 oder aus einem der Puffer-Trays 125 austauschen.

Um ein Verrutschen des Eier-Trolleys 80 zu verhindern, ist in dem in Fig. 11 gezeigten Ausführungsbeispiel eine Trolley-Fixierung 120 vorgesehen, welche den Eier-Trolley 80 mit der mobilen Messeinheit 122, lösbar verbindet.

Der Vorteil des beschriebenen Ausführungsbeispiels liegt darin, dass die mobile Messeinheit 122 in einem geschlossenen Inkubator "geparkt" werden kann, und dann selbstständig die zu messenden Eier-Trays 40 in leere Puffer-Trays 125 überführt, um die Messung durchzuführen. Dadurch wird vermieden, dass die Inkubatortür 105 (nicht gezeigt) zu oft geöffnet werden muss, wodurch sich nachteilige Temperaturschwankungen ergeben können. Gleichzeitig kann die mobile Messeinheit 122 nach Bedarf zwischen verschiedenen Inkubatoren hin und her bewegt werden.

Allen obigen Ausführungsbeispielen ist gemein, dass die Auswertungseinheit dazu ausgebildet ist, anhand des gemessenen Spektrums eines Eis 50 ein Geschlechtslabel auszugeben und eine Konfidenz des Geschlechtslabels zu bestimmen und auszugeben. Die Konfidenz gibt dabei eine geschätzte Wahrscheinlichkeit an, mit der das Geschlechtslabel korrekt ist.

Anhand der Konfidenz kann zudem entschieden werden, ein bestimmtes oder mehrere Eier 50 weiteren Messungen zu unterziehen um damit anhand der vorhandenen Statistik die Konfidenz über einen gewünschten Wert zu heben. Gleichzeitig kann auch der Beobachtungszeitraum für Eier 50 auf Basis der Konfidenz verlängert werden, um diese zu erhöhen.

Ein Spektrum kann dabei aus beliebig vielen, eventuell gewichteten, Einzelmessungen zusammengesetzt werden. Die Verwendung von mehreren Messungen, beispielsweise von 10 oder mehr, ermöglicht dabei eine bessere Statistik und somit eine höhere Genauigkeit des Spektrums, was wiederum Einfluss auf die Konfidenz der resultierenden Geschlechtslabels hat. Um die Dauer eines Messvorgangs dabei gering zu halten, dauert eine Einzelmessung dabei vorzugsweise nicht länger als 60 µs, weiter vorzugsweise weniger als 40 µs, noch weiter vorzugsweise weniger als 20 µs.

Gemäß der vorliegenden Erfindung wird bei der Messung ein Winkel zwischen einer absoluten Referenzachse, beispielsweise der Vertikalen, und der optischen Messachse bestimmt. Die Neigung der Messachse bzw. der Kippwinkel kann zum Beispiel durch Auslesen der Motor-Position des Kipp-Mechanismus, durch einen Hall-Sensor oder Potentiometer an der Kipp-Achse des Eier-Trays oder durch Messen des Abstands zwischen der Außenkante des untersten Eier-Trays und eines fixen Referenzpunktes erfolgen. Nachdem der Embryo im Verhältnis zur Richtung der Schwerkraft immer oben aufschwimmt, kann die Veränderung des Winkels verwendet werden um die Lage des Embryos innerhalb der Messanordnung zu beeinflussen.

Insbesondere können zur besseren Auswertung mehrere Messungen gemacht werden, welche verschiedenen Positionen des Embryos entsprechen. Beispielsweise ist es denkbar, dass zum Erzeugen eines Referenzspektrums der Embryo aus dem Sichtfeld der Sensoreinheit "herausgekippt" wird, um eine Messung ohne Embryo als Referenz zu haben. Wichtig ist hierbei, dass zwischen Messungen bei verschiedenen Kippwinkeln lange genug gewartet wird, um es dem Embryo zu ermöglichen, in eine Ruheposition bzw. einen Gleichgewichtszustand zu kommen. Dies verhindert, dass der Embryo unbeabsichtigt die Position während einer Messung ändert. Wird eine Bestrahlungseinheit mit einer Vielzahl von Strahlungsquellen verwendet, wie obenstehend in Bezug auf Fig. 2a-2c beschrieben wurde, können ferner Messungen unter Verwendung verschiedener Strahlungsquellen durchgeführt werden, um die unterschiedlichen Positionen der Embryos in den Eiern besser zu berücksichtigen. Das Konzept mehrerer Messungen mit unterschiedlichen aktivierten Lichtquellen kann mit dem Konzept der Einstellung des Kippwinkels kombiniert werden, um die Qualität der Messungen weiter zu verbessern.

Anhand der gespeicherten Referenzspektren kann eine Normalisierung der Messspektren erfolgen. Dabei wird beispielsweise das Signal des Referenzspektrums von dem eines Messspektrums abgezogen, um die tatsächliche Veränderung zu erfassen und Abweichungen, welche durch Variationen des Eis selbst entstehen, herauszufiltern. Um eine möglichst gute Referenzfunktion zu gewährleisten, ist es dabei von Vorteil, die Referenzmessung in einem sehr frühen Stadium der embryonalen Entwicklung durchzuführen. Insbesondere ist für einen guten Referenzwert vorteilhaft, die Referenzmessung bereits vor Beginn der Inkubation zu nehmen.

Es kann ferner vorgesehen sein, die Sensoreinheit durch Eichmessungen im Betrieb zu kalibrieren. Dazu werden üblicherweise Referenzobjekte so wie beispielsweise Teflon-Blöcke verwendet, da diese das Spektrum der Strahlungsquelle in bekannter Weise beeinflussen und somit Rückschlüsse auf etwaige Messfehler erlauben. In denjenigen Ausführungsbeispielen in welchen die Bestrahlungseinheit 10 und Sensoreinheit 20 mobil ausgestaltet wird, kann dabei eine Automatisierung solcher Eichmessungen erfolgen, indem das Messsystem in festgelegten Intervallen Messungen an Referenzobjekten durchführt.

Ebenso ist es denkbar, in festgelegte Trolleys 80 Referenzobjekte auf ein Eier-Tray 40 zu setzen, um die Eichmessungen mit Hilfe der Trolley-Transportvorrichtung 81 zu automatisieren. Im Fall eines Mess-Aufsatzes 102 wird die Sensoreinheit vor dem Aufsetzen mit Hilfe eines Referenz-Trays kalibriert.

Eichmessungen können dabei ebenfalls Messungen umfassen, bei denen die Sensoreinheit absichtlich abgedeckt wird, um das "dark noise" des Spektrometers zu überprüfen.

Die für die Geschlechtsermittlung herangezogenen Wellenlängenbereiche können hierbei im Absorptionsbereich von Hämoglobin liegen, also zwischen 500 nm und 900 nm, sind aber nicht hierauf beschränkt.

Die Auswertung der gemessenen Daten kann dabei direkt auf der Datenverarbeitungseinheit 33 stattfinden. Eine endgültige Klassifizierung erfolgt durch die potentiell ausgelagerte Klassifikationseinheit 35.

Die Klassifikationseinheit 35 erhält dabei die vorbearbeiteten Messdaten der Datenverarbeitungseinheit 33. Zusätzlich greift die Klassifikationseinheit 35 auf extern gespeicherte Daten zu, welche bei der Klassifikation der Eier berücksichtigt werden.

Insbesondere können im erfindungsgemäßen Verfahren über das reine Spektrum hinaus sogenannte Zusatzdaten bei der Klassifikation oder bei der Bestimmung der Konfidenz berücksichtigt werden. Beispiele hierfür sind unter anderem die Ei-Größe (Durchmesser, Höhe), Ei-Form, Ei-Gewicht, Ei-Farbe, Lagerungsdauer seit Schlupf, Alter der Elterntiere, Tier-Rasse, Herkunft, Ei-Orientierung, Orientierung der Luftblase im Ei oder Hinweise auf Beschädigungen am Ei. Die Zusatzdaten werden dabei von der Datenverarbeitungseinheit 33, bzw. der Klassifikationseinheit 35, empfangen und fließen in die Bestimmung des Geschlechts oder der Konfidenz ein. Beispielsweise kann bei suboptimalen Ei-Orientierungen oder bei Beschädigungen des Eis die Konfidenz herab- oder heraufgesetzt werden. Auch können Eier, welche Beschädigungen aufweisen, grundsätzlich einem (nicht bevorzugten) Geschlecht zugeordnet werden, um sie später auszusortieren.

Die Zusatzdaten können dabei weiterhin auch weitere Informationen, wie beispielsweise die Sensortemperatur/-feuchtigkeit zu mindestens einem Zeitpunkt, die Inkubatortemperatur /-feuchtigkeit zu mindestens einem Zeitpunkt oder Störungsmeldungen aus dem Inkubationsprozess umfassen.

Zusätzlich können Zusatzdaten auch regulatorische Vorgaben oder manuell definierte Klassifikationsregeln und oder Aussortierungsregeln enthalten. Diese erlauben eine Berücksichtigung der geplanten maximalen oder minimalen Outputmenge bei der Klassifikation. Beispielsweise ist es vorgesehen, bei sich abzeichnender Unterschreitung einer minimalen Outputmenge eines Geschlechts die Anforderungen hinsichtlich der Konfidenz für eine Klassifizierung in besagtes Geschlecht herabzusetzen, um zu garantieren, dass genug Output vorhanden ist. Dabei können die Zusatzdaten also insbesondere auch die Klassifikationsergebnisse oder Konfidenzergebnisse von anderen Eiern umfassen, um einen geeigneten Erwartungswert für den Gesamtoutput zu erreichen.

Die Speicherung der Zusatzdaten kann dabei dezentral in einer Cloud oder lokal in der Datenverarbeitungseinheit 33 erfolgen.

Dies ermöglicht eine genaue Einstellung von externen Parametern, wie zum Beispiel die gewünschte Geschlechtsverteilung, Mindestquoten für einzelne Geschlechter, gewünschte Outputmenge etc., wenn über eine Aussortierung von Eiern anhand des Geschlechtslabels und der Konfidenz entschieden wird. Dabei können auch weiter bekannte, geschätzte oder festgestellte Parameter, wie beispielsweise die Sterberate von Embryos, berücksichtigt werden.

### Bezugszeichenliste:

- 10: Bestrahlungseinheit
- 11: Lichtquelle (LED)
- 12: (LED-)Kühlelement
- 13: optisches Lenkelement (asphärische oder Fresnel-Linse)
- 14: Strahlbegrenzer

- 20: Sensoreinheit
- 21: optisches Sammelelement (Kollimationslinse)
- 22: Lichtwellenleiter
- 23: Identifikationseinheit (Identifikations- und Positionsbestimmungseinheit)
- 24: optisches Entkopplungselement (Dichtring, bislang nur in Fig. 2 zu sehen)

- 30: Auswertungseinheit
- 31: Spektrometer
- 32: Kühlelement
- 33: Datenverarbeitungseinheit
- 34: Anschlüsse (Strom- und Datenkabel)
- 35: Klassifikationseinheit

- 40: (integriertes) Eier-Tray
- 41: Eier-Tray-Auflage
- 42: Eierausnehmung
- 43: optisches Entkopplungselement (Gummierung)
- 44: Eier-Tray-Identifikationsmerkmal (ID Tag)
- 45: Befestigungsmittel

- 50: Ei
- 51: Lufteinschluss
- 52: Blutgefäße

- 60: erster Messarm
- 61: erster Bewegungsmechanismus

- 70: zweiter Messarm
- 71: zweiter Bewegungsmechanismus

- 80: Eier-Trolley
- 81: Trolley-Transportvorrichtung
- 82: Trolley-Kippvorrichtung
- 83: Trolley-Führungselement
- 84: Trolley-Fuß
- 85: Trolley-Positionierungsvorrichtung
- 86: Kippmechanismus

- 90: Messsäule (für Messarme mit Linearführung)
- 91: Säulenfuß
- 92: erste Horizontal-Linearführung
- 93: zweite Horizontal-Linearführung
- 94: Messarm
- 101: Eier-Fixierungsselement
- 102: Messaufsatz
- 103: Messaufsatz-Adapter
- 104: Inkubatorwand
- 105: Inkubatortür
- 106: Belüftungsöffnungen
- 107: Transportmittel
- 110: Messkopfgehäuse
- 111: Erste Konvexlinse
- 112: Zweite Konvexlinse
- 113: Fokussierter Lichtkegel
- 114: Diffuser Lichtstrahl

- 120: Trolley-Fixierung
- 122: Mobile Messeinheit
- 125: Puffer-Tray
- 127: Tray-Schlitten

## Patentansprüche

1. Messsystem zur nicht-invasiven, vorzugsweise automatisierten, Geschlechtserkennung von Embryos in einem Ei (50) in der früh-embryonalen Entwicklung, insbesondere vor dem siebten Bruttag, insbesondere während der Inkubation, insbesondere in einem Inkubator mit mindestens einem Eier-Tray (40) zur Aufnahme einer Vielzahl von Eiern (50) und mindestens einem Eier-Trolley (80) zur Halterung mindestens eines Eier-Trays (40), aufweisend:
- mindestens eine Bestrahlungseinheit (10) zur Bestrahlung eines Eis (50) mit elektromagnetischer Strahlung;
- mindestens eine Sensoreinheit (20) zur Erfassung von durch das Ei (50) transmittierter elektromagnetischer Strahlung;
- eine Auswertungseinheit (30) mit
∘ mindestens einem Spektrometer (31), das dazu ausgebildet ist, die durch das Ei (50) transmittierte Strahlung zu empfangen und ein Spektrum der durch das Ei (50) transmittierten Strahlung zu erzeugen; und
∘ einer Datenverarbeitungseinheit (33), die dazu ausgebildet ist, von dem Spektrometer (31) erzeugte Spektren zu empfangen und als Referenzspektrum oder Messspektrum abzuspeichern;
- eine Identifikationseinheit (23) zur Erzeugung von Identifikationsdaten, mittels derer die an einem Ei (50) erzeugten Spektren dem Ei (50) eindeutig zuordenbar sind;
- eine Klassifikationseinheit (35);
wobei die Datenverarbeitungseinheit (33) dazu ausgebildet ist, von dem Spektrometer (31) erzeugte Spektren und zugehörige Identifikationsdaten zu speichern, und wobei die Klassifikationseinheit (35) dazu ausgebildet ist, das Geschlecht des Embryos auf Grundlage mindestens eines Referenzspektrums und mindestens eines Messspektrums zu bestimmen.

2. Messsystem nach Anspruch 1, aufweisend ein optisches Entkopplungselement (43) zur optischen Entkopplung der Bestrahlungseinheit (10) und der Sensoreinheit (20), das vorzugsweise dazu ausgebildet ist, während einer Messung an dem Ei (50) anzuliegen.

3. Messsystem nach Anspruch 1 oder 2, aufweisend Mittel zur Bestimmung eines Winkels zwischen einer Referenzachse des Eis (50) und einer Referenzachse des Messsystems.

4. Messsystem nach einem der vorhergehenden Ansprüche, wobei die Bestrahlungseinheit (10) eine Vielzahl von Strahlungsquellen (10a) aufweist, und vorzugsweise als Ringleuchte ausgebildet ist, bei der die Vielzahl von Strahlungsquellen (10a) auf einem Ring angeordnet ist.

5. Messsystem gemäß einem der vorhergehenden Ansprüche, wobei die Bestrahlungseinheit (10) und die Sensoreinheit (20) derart angeordnet und konfiguriert sind, dass sie bei jeder Messung relativ zu einem Bezugspunkt des Eis (50), beispielsweise zum Schwerpunkt der Schale des Eis (50), dieselbe Ausrichtung zueinander haben.

6. Messsystem nach einem der vorhergehenden Ansprüche, aufweisend eine Trolley-Transportvorrichtung (81) zum Transport eines Eier-Trolleys (80) mit mindestens einem Eier-Tray (40) zu mindestens einer Bestrahlungseinheit (10), die vorzugsweise innerhalb des Inkubators angeordnet ist, und/oder eine Trolley-Positionierungsvorrichtung (85), welche dazu geeignet ist, eine Position des Trolleys (80) innerhalb des Inkubators eindeutig festzulegen.

7. Messsystem nach einem der vorhergehenden Ansprüche, aufweisend Transportmittel (107) zum Transport der Bestrahlungseinheit (10) und der Sensoreinheit (20) zu einem Ei (50), vorzugsweise innerhalb des Inkubators, weiter vorzugsweise zwischen verschiedenen Inkubatoren.

8. Messsystem nach einem der vorhergehenden Ansprüche, insbesondere nach Anspruch 6, wobei die Trolley-Transportvorrichtung (81) Mittel aufweist, die dazu ausgebildet sind, eine Kippstellung mindestens eines Eis (50) einzustellen.

9. Messsystem nach einem der vorhergehenden Ansprüche, aufweisend einen Messarm (94), welcher die Bestrahlungseinheit (10) und die Sensoreinheit (20) derart aufnimmt, dass ein Ei (50) zur Messung an der Bestrahlungseinheit (10) und der Sensoreinheit (20) positionierbar ist.

10. Messsystem nach einem der vorhergehenden Ansprüche, aufweisend einen ersten Messarm (60), welcher die Bestrahlungseinheit (10) aufnimmt und einen zweiten Messarm (70), welcher die Sensoreinheit (20) aufnimmt, wobei der erste Messarm (60) und der zweite Messarm (70) derart angeordnet und ausgebildet sind, dass ein Ei zur Messung zwischen der Bestrahlungseinheit (10) und der Sensoreinheit (20) positionierbar ist.

11. Messsystem nach Anspruch 10, aufweisend eine Messsäule (90), an der der erste Messarm (60) und der zweite Messarm (70) beweglich gelagert sind, wobei der erste Messarm (60) einen ersten Bewegungsmechanismus (61) zur Einstellung einer Vertikalposition des ersten Messarms aufweist,
wobei der zweite Messarm (70) einen zweiten Bewegungsmechanismus (71) zur Einstellung einer Vertikalposition des zweiten Messarms aufweist,
wobei der erste Bewegungsmechanismus (61) vorzugsweise eine erste Horizontal-Linearführung (92) und eine zweite Horizontal-Linearführung (93) zur Einstellung einer Horizontalposition des ersten Messarms (60) aufweist, und
wobei der zweite Bewegungsmechanismus (71) vorzugsweise eine dritte Horizontal-Linearführung (92) und eine vierte Horizontal-Linearführung (93) zur Einstellung einer Horizontalposition des zweiten Messarms (70) aufweist.

12. Messsystem nach einem der vorhergehenden Ansprüche, wobei die Auswertungseinheit (30) dazu ausgebildet ist, für jedes Ei (50) ein Geschlechtslabel und eine zugehörige Konfidenz auszugeben.

13. Messsystem nach einem der vorhergehenden Ansprüche, aufweisend einen extern angebundenen Datenspeicher, insbesondere einen Cloud-Speicher, der dazu konfiguriert ist, externe Parameter wie Sterberate der Embryos oder gewünschte Outputmenge, Mess- und Referenzspektren, und/oder Ergebnisse der Auswertung des Messsystems zu speichern und an die Klassifikationseinheit (35) auszugeben.

14. Messsystem nach einem der vorhergehenden Ansprüche, umfassend Befestigungsmittel (45), welche dazu ausgebildet sind, eine Änderung der Ausrichtung des Eis (50) zu verhindern.

15. Messsystem nach einem der vorhergehenden Ansprüche, insbesondere nach Anspruch 13, wobei die Klassifikationseinheit (35) dazu ausgebildet ist, anhand der Daten der Messeinheit (20) und/oder aus dem extern angebundenen Speicher eine Klassifikation eines Eis (50) nach Geschlecht und/oder Gesundheitszustand vorzunehmen, wobei die Klassifikationseinheit (35) vorzugsweise räumlich vom Rest der Auswertungseinheit (30) getrennt ist, und insbesondere vorzugsweise durch eine Software-Komponente auf einem externen Server, vorzugsweise auf einem Cloud Server, gebildet ist.
